(19) Europäisches Patentamt
European Patent Office
Office européen des brevets

(11) Veröffentlichungsnummer: **0 259 254 B1**

(12) # EUROPÄISCHE PATENTSCHRIFT

(45) Veröffentlichungstag der Patentschrift: **17.04.91**

(51) Int. Cl.[5]: **C07D 277/70**, C09D 5/08, C10M 135/36

(21) Anmeldenummer: **87810468.6**

(22) Anmeldetag: **21.08.87**

Die Akte enthält technische Angaben, die nach dem Eingang der Anmeldung eingereicht wurden und die nicht in dieser Patentschrift enthalten sind.

(54) **Phenolische Benzthiazolderivate und ihre Verwendung als Korrosionsinhibitoren.**

(30) Priorität: **27.08.86 GB 8620668**

(43) Veröffentlichungstag der Anmeldung:
**09.03.88 Patentblatt 88/10**

(45) Bekanntmachung des Hinweises auf die Patenterteilung:
**17.04.91 Patentblatt 91/16**

(84) Benannte Vertragsstaaten:
**AT BE DE ES FR GB IT NL SE**

(56) Entgegenhaltungen:
**EP-A- 0 003 817**
**SU-A- 1 164 233**

**Chemical Abstracts, Band 83, Nr. 1, 7 Juli 1975, Columbus, Ohio, USAV.G. Sorokin "Synthesis of 3-(2-hydroxyethyl)-and 3-(bis(2-hydroxyethyl)) aminomethylbenzothiazolyl-2-thiones and some of their transformations" Seite 825, Spalte 1, Zusammenfassung-Nr. 9 878p**

**Derwent Accession Nr. 83-40 141 Questel Telesystemes (WPIL) Derwent Publications Ltd. London**

(73) Patentinhaber: **CIBA-GEIGY AG**
**Klybeckstrasse 141**
**CH-4002 Basel(CH)**

(72) Erfinder: **Braig, Adalbert, Dr.**
**Zollstrasse 1/1**
**W-7858 Weil-Friedlingen(DE)**
Erfinder: **Meier, Hans-Rudolf, Dr.**
**Route du Confin 54**
**CH-1723 Marly(CH)**
Erfinder: **Leppard, David G., Dr.**
**Route de Bourguillon 6**
**CH-1723 Marly(CH)**
Erfinder: **Wasson, Robert Craig, Dr.**
**27 Phythian Crescent Penketh**
**Warrington Cheshire WA5 2BT(GB)**
Erfinder: **Phillips, Emyr, Dr.**
**12 Pinwood Court Broad Road**
**Sale Cheshire M33 2ES(GB)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft phenolische Derivate des Benzthiazols und ihre Verwendung als Korrosionsinhibitoren in organischen Materialien, insbesondere in Anstrichstoffen und in Schmiermitteln.

Mercaptobenzthiazol und seine Salze sind als Korrosionsinhibitoren bekannt, z.B. aus der EP-A-3817. Es wurden auch bereits verschiedene Derivate des Mercaptobenzthiazols als Korrosionsinhibitoren vorgeschlagen, z.B. die von der EP-A_129.506 beschriebenen Benzthiazol-2-ylthiocarbonsäuren und deren Salze. Hierbei handelt es sich vorwiegend um Derivate mit hydrophilen Gruppen.

Es wurde nunmehr gefunden, dass auch bestimmte Benzthiazolderivate mit hydrophoben Gruppen ausgezeichnete Korrosionsinhibitoren sein können. Diese Verbindungen wirken ausserdem als Antioxidantien und Lichtschutzmittel. Sie sind daher als Zusätze für solche organische Materialien verwendbar, bei denen eine Korrosionsinhibierung und/oder eine antioxidative Stabilisierung oder UV-Stabilisierung gewünscht wird. Dies ist insbesondere der Fall bei Anstrichstoffen und bei Schmiermitteln.

Die Verbindungen zeichnen sich gegenüber bekannten Korrosionsinhibitoren auf Basis von Benzthiazolderivaten durch geringere Wasseraufnahme, durch chemische Inertheit und durch eine hohe thermische Stabilität aus.

Die Erfindung betrifft vor allem Zusammensetzungen aus einem Anstrichstoff oder einem Schmiermittel, enthaltend mindestens eine Verbindung der Formel I,

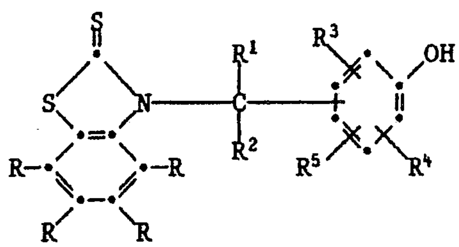

I

worin jedes R unabhängig voneinander Wasserstoff, $C_1$-$C_{12}$-Alkyl, $C_1$-$C_4$-Halogenalkyl, $C_1$-$C_{12}$-Alkoxy, $C_1$-$C_{12}$-Alkylthio, Phenylthio, Benzylthio, $C_1$-$C_{12}$-Alkylsulfonyl, Phenyl, $C_7$-$C_{15}$-Alkylphenyl, $C_7$-$C_{10}$-Phenylalkyl, $C_5$-$C_8$-Cycloalkyl, Halogen, $-NO_2$, -CN, -COOH, -COO($C_1$-$C_4$-Alkyl), -OH, $-NH_2$, $NHR^6$, $-N(R^6)_2$, $-CONH_2$, $-CONHR^6$ oder $-CON(R^6)_2$ bedeutet,

$R^1$ Wasserstoff, $C_1$-$C_{12}$-Alkyl, Phenyl, durch Halogen, $C_1$-$C_4$-Alkyl, $C_1$-$C_4$-Alkoxy oder $-NO_2$ substituiertes Phenyl, Pyridyl, Thienyl oder Furyl bedeutet,

$R^2$ Wasserstoff oder $C_1$-$C_4$-Alkyl bedeutet,

$R^3$ und $R^4$ unabhängig voneinander Wasserstoff, Halogen, $C_1$-$C_4$-Alkoxy, Cyano, Nitro, $C_1$-$C_{20}$-Alkyl, --$(CH_2)_m$-$COOR^7$ -$(CH_2)_m$-$CONHR^6$, $(CH_2)_m$-$CON(R^6)_2$, $C_3$-$C_{20}$-Alkenyl, $C_7$-$C_{10}$-Phenylalkyl, Phenyl, Cyclohexyl, Cyclopentyl oder eine Gruppe der Formel II

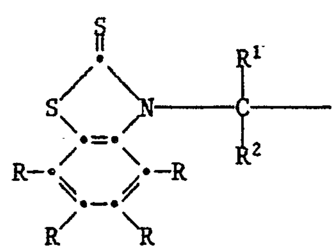

II

bedeuten,

$R^5$ Wasserstoff, $C_1$-$C_{20}$-Alkyl, $C_3$-$C_{20}$-Alkenyl oder Hydroxy bedeutet, oder $R^3$ und $R^5$ zusammen oder $R^4$ und $R^5$ zusammen einen Ring bilden, der am Phenolring anelliert ist und der ein carbocyclischer oder heterocyclischer Ring sein kann, welcher als Heteroatome Sauerstoff, Stickstoff oder Schwefel enthalten kann und welcher durch $C_1$-$C_4$-Akyl, $C_1$-$C_4$-Alkoxy oder Halogen substituiert sein kann,

$R^6$ $C_1$-$C_{12}$-Alkyl, durch ein oder mehrere O-Atome unterbrochenes $C_3$-$C_{12}$-Alkyl, $C_5$-$C_8$-Cycloalkyl, Benzyl, Phenyl oder durch Halogen, $C_1$-$C_4$-Alkyl, $C_1$-$C_4$-Alkoxy oder Nitro substituiertes Phenyl bedeutet oder $-N(R^6)_2$ eine Pyrrolidino-, Piperidino- oder Morpholinogruppe bedeutet,

$R^7$ Wasserstoff oder $C_1$-$C_{20}$-Alkyl bedeutet, das durch Halogen oder Hydroxyl substituiert sein kann, oder $C_3$-$C_{20}$-Alkyl bedeutet, das durch eine oder mehrere Sauerstoffatome unterbrochen ist und durch Hydroxyl

substituiert sein kann, und m O, 1 oder 2 bedeutet.

In Formel I können R, $R^1$, $R^2$, $R^3$, $R^4$, $R^5$, $R^6$ oder $R^7$ als Alkyl unverzweigtes oder verzweigtes Alkyl sein. Soweit dies $C_1$-$C_4$-Alkyl ist, können dies z.B. Methyl, Ethyl, n-Propyl, Isopropyl, n-Butyl, sec.Butyl, Isobutyl oder tert.Butyl sein. R, $R^1$, $R^2$, $R^3$, $R^4$, $R^5$, $R^6$ und $R^7$ können auch $C_5$-$C_{12}$-Alkyl sein, wie z.B. Pentyl, Hexyl, n-Octyl, 2-Ethylhexyl, 1,1,3,3-Tetramethylbutyl, 1,1,3,3,5,5-Hexamethylhexyl, n-Decyl, Isodecyl oder n-Dodecyl. $R^3$, $R^4$, $R^5$ und $R^7$ können auch $C_{13}$-$C_{20}$-Alkyl sein, wie z.B. Tridecyl, Tetradecyl, Pentadecyl, Hexadecyl, Octadecyl oder Eikosyl.

$R^3$, $R^4$ und $R^5$ als $C_3$-$C_{20}$-Alkenyl können z.B. Allyl, Methallyl, 2-Butenyl, 2-Hexenyl, Decenyl, Undecenyl, Pentadecenyl oder Octadecenyl (Oleyl) sein.

R als Halogenalkyl kann z.B. Chlormethyl, Trichlormethyl, Brommethyl, 2-Chlorethyl, 2,2,2-Trichlorethyl, Trifluormethyl oder 2,3-Dichlorpropyl sein.

R als Alkoxy, Alkylthio oder Alkylsulfonyl kann z.B. Methoxy, Ethoxy, Isopropoxy, Butoxy, Hexyloxy, Octyloxy, Dodecyloxy, Methylthio, tert.Butylthio, Dodecylthio, Methylsulfonyl, Ethylsulfonyl, Hexylsulfonyl oder Dodecylsulfonyl sein. R als Alkylphenyl kann z.B. Tolyl, Xylyl, 4-Ethylphenyl, 4-tert.Butylphenyl, 4-Octylphenyl oder 4-Nonylphenyl sein.

R, $R^3$ und $R^4$ als Phenylalkyl können z.B. Benzyl, 1-Phenylethyl, 2-Phenylethyl, $\alpha$, $\alpha$-Dimethylbenzyl oder 2-Phenylpropyl sein.

R und $R^6$ als Cycloalkyl können z.B. Cyclopentyl, Cyclohexyl, Cycloheptyl, Methylcyclohexyl oder Cyclooctyl sein.

$R^1$ und $R^6$ als durch Halogen, $C_1$-$C_4$-Alkyl, $C_1$-$C_4$-Alkoxy oder Nitro substituiertes Phenyl können z.B. 4-Chlorphenyl, 3-Bromphenyl, 2-Fluorphenyl, p-Tolyl, 3,5-Dimethylphenyl, 4-Isopropylphenyl, 4-Methoxyphenyl, 3-Ethoxyphenyl, 4-Nitrophenyl oder 4-Nitro-2-methylphenyl sein.

$R^6$ und $R^7$ als durch O unterbrochenes Alkyl kann z.B. 2-Methoxyethyl, 2-Butoxyethyl, 3,6-Dioxaheptyl oder 3,6-Dioxadecyl sein. $R^7$ kann auch ein Polyethylenglykolrest mit bis zu 20 C-Atomen und bis zu 10 O-Atomen sein.

Wenn $R^3$ und $R^5$ zusammen oder $R^4$ und $R^5$ zusammen einen annelierten Ring bilden, so kann dies insbesondere ein Benzolring, ein Pyridinring oder ein Benzofuranring sein. Unter Einschluss des Phenolringes entstehen dann ein Naptholrest, ein Hydroxychinolinrest oder ein Hydroxydibenzofuranrest.

Bevorzugt sind Zusammensetzungen enthaltend eine Verbindung der Formel I, worin in Formel I und II ein R Wasserstoff, $C_1$-$C_4$-ALkyl, $C_1$-$C_4$-Alkoxy, Trifluormethyl, Halogen oder Nitro bedeutet und die anderen drei R Wasserstoff sind.

Bevorzugt sind weiterhin Zusammensetzungen enthaltend eine Verbindung der Formel I, worin $R^1$ Wasserstoff, $C_1$-$C_8$-Alkyl, Phenyl oder Furyl bedeutet und $R^2$ Wasserstoff ist, insbesondere eine Verbindung der Formel I, worin $R^1$ und $R^2$ Wasserstoff sind.

Bevorzugt sind weiterhin Zusammensetzungen enthaltend eine Verbindung der Formel I, worin $R^3$ und $R^4$ unabhängig voneinander Wasserstoff, $C_1$-$C_8$-Alkyl, Allyl, $C_7$-$C_{10}$-Phenylalkyl, $C_1$-$C_4$-Alkoxy, Halogen, Phenyl, Cyclohexyl oder eine Gruppe $-CH_2CH_2COOR^7$ bedeuten, $R^5$ Wasserstoff, $C_1$-$C_{18}$-Alkyl, $C_3$-$C_{18}$-Alkenyl oder OH ist und $R^7$ $C_1$-$C_{18}$-Alkyl oder durch ein oder mehreren Sauerstoffatome unterbrochenes $C_3$-$C_{20}$-Alkyl bedeutet.

In Formel I steht die phenolische OH-Gruppe bevorzugt in Para- oder Orthoposition zur Gruppe

$$>C(R^1)(R^2).$$

Wenn sie in Paraposition steht, so sind Verbindungen der Formell III bevorzugt,

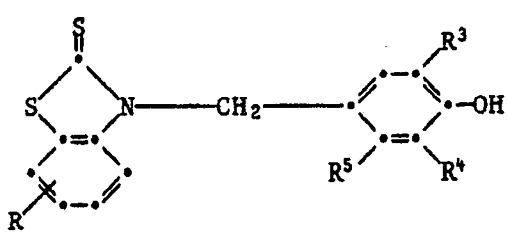

III

worin R Wasserstoff, $C_1$-$C_4$-Alkyl, $C_1$-$C_4$-Alkoxy, Chlor, Trifluormethyl oder Nitro ist, $R^3$ und $R^4$ unabhängig voneinander Wasserstoff, $C_1$-$C_8$-Alkyl, Allyl, Chlor, Methoxy, $C_7$-$C_{10}$-Phenylalkyl, Phenyl oder Cyclohexyl sind, und $R^5$ H, $CH_3$ oder OH ist.

Wenn in Formel I die phenolische OH-Gruppe in Orthostellung steht, so sind Verbindungen der Formel

IV bevorzugt,

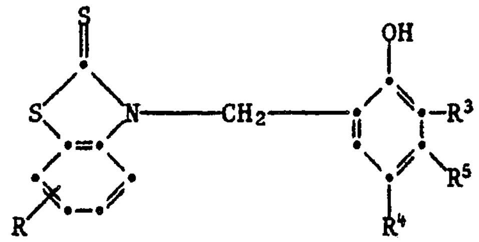

IV

worin R Wasserstoff, $C_1$-$C_4$-Alkyl, Chlor, Trifluormethyl oder Nitro ist,
$R^3$ und $R^4$ unabhängig voneinander Wasserstoff, $C_1$-$C_8$-Alkyl, $C_7$-$C_{10}$-Phenylalkyl, Phenyl, Cyclohexyl oder eine Gruppe der Formel IIa sind

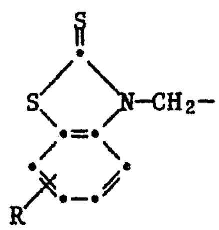

IIa

oder $R^3$ und $R^4$ zusammen einen anellierten Benzolring, Pyridinring oder Benzofuranring bilden.

Beispiele für einzelne Verbindungen der Formel III sind solche mit den im folgenden angegebenen Substituenten:

| Verbindung Nr. | R | $R^3$ | $R^4$ | $R^5$ |
|---|---|---|---|---|
| 1 | H | t-Butyl | t-Butyl | H |
| 2 | H | t-Butyl | Methyl | H |
| 3 | H | Phenyl | Phenyl | H |
| 4 | H | Methyl | Methyl | H |
| 5 | H | Methyl | Cyclohexyl | H |
| 6 | H | Cyclohexyl | Cyclohexyl | H |
| 7 | H | Phenyl | t-Butyl | H |
| 8 | H | Methoxy | Methoxy | H |
| 9 | H | Chlor | Chlor | H |
| 10 | H | Isopropyl | Isopropyl | H |
| 11 | H | 1-Methylpropyl (sec.Butyl) | 1-Methylpropyl | H |
| 12 | H | Methyl | Ethyl | H |
| 13 | H | 1-Phenylethyl | 1-Phenylethyl | H |
| 14 | H | α,α-Dimethylbenzyl | α,α-Dimethylbenzyl | H |
| 15 | H | 1-Methylheptyl (sec.Octyl) | 1-Methylheptyl | H |
| 16 | H | Methyl | tert.Butyl | Methyl |
| 17 | H | Methyl | Tetramethylbutyl | Methyl |
| 18 | 4-Cl | 1,1-Dimethylpropyl (tert.Amyl) | 1,1-Dimethylpropyl | H |
| 19 | 5-$NO_2$ | 2-Methylpropyl (Isobutyl) | 2-Methylpropyl | H |
| 20 | 5-$CF_3$ | t-Butyl | t-Butyl | H |
| 21 | H | Methyl | Methyl | Methyl |
| 22 | 5-$NO_2$ | t-Butyl | t-Butyl | H |
| 23 | H | Cyclohexyl | t-Butyl | H |
| 24 | H | Phenyl | Methyl | H |
| 25 | 5-Cl | t-Butyl | t-Butyl | H |
| 26 | 6-$C_2H_5O$ | t-Butyl | t-Butyl | H |
| 27 | H | H | H | H |
| 28 | H | Methyl | Allyl | H |
| 29 | H | Isopropyl | Isopropyl | OH |

Beispiele für einzelne Verbindungen der Formel IV sind solche mit den folgenden Substituenten:

| Verbindung Nr. | R | $R^3$ | $R^4$ | $R^5$ |
|---|---|---|---|---|
| 30 | H | t-Butyl | Methyl | H |
| 31 | H | t-Butyl | t-Butyl | H |
| 32 | H | 1,1,3,3-Tetramethylbutyl | 1,1,3,3-Tetramethylbutyl | H |
| 33 | H | 1-Methylpropyl | t-Butyl | H |
| 34 | H | 1,1-Dimethylbutyl (t-Hexyl) | 1,1-Dimethylbutyl | H |
| 35 | H | H | Methyl | H |
| 36 | H | Methyl | Methyl | H |
| 37 | H | Isopropyl | Isopropyl | H |
| 38 | H | Isopropyl | Methyl | H |
| 39 | 5-Cl | t-Hexyl | Methyl | H |
| 40 | H | Methyl | t-Hexyl | H |
| 41 | 4-$CH_3$ | t-Butyl | Isopropyl | H |
| 42 | H | t-Hexyl | Isopropyl | H |
| 43 | H | Methyl | α,α-Dimethylbenzyl | H |
| 44 | 5-$NO_2$ | α,α-Dimethylbenzyl | Methyl | H |
| 45 | H | Ethyl | α,α-Dimethylbenzyl | H |
| 46 | H | t-Butyl | α,α-Dimethylbenzyl | H |
| 47 | H | α,α-Dimethylbenzyl | α,α-Dimethylbenzyl | H |
| 48 | H | 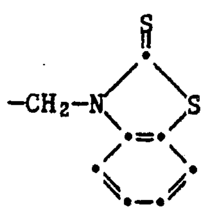 | Methyl | H |
| 49 | H | Methyl | 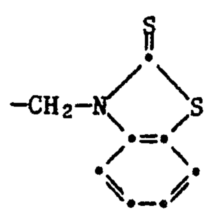 | H |
| 50 | H | t-Amyl | t-Amyl | H |
| 51 | H | sec. Butyl | t-Amyl | |

| Verbindung Nr. | R | $R^3$ | $R^4$ | $R^5$ |
|---|---|---|---|---|
| 52 | H | H | H | t-Butyl |
| 53 | H | H | H | Pentadecyl |
| 54 | H | Methyl | Allyl | H |
| 55 | H | H | H | Pentadecenyl |
| 56 | H | sec. Butyl | sec. Butyl | H |
| 57 | H | Cyclohexyl | t-Butyl | H |
| 58 | 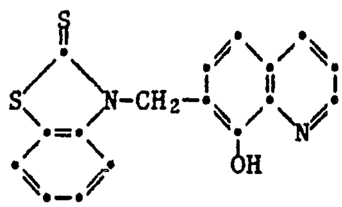 | | | |
| 59 | 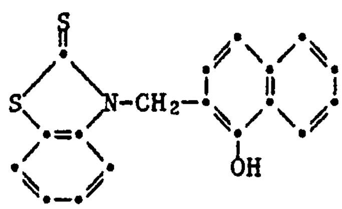 | | | |
| 60 | 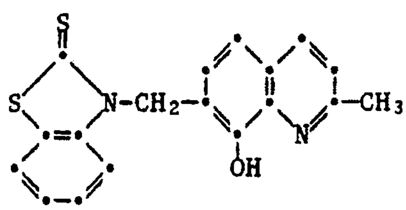 | | | |
| 61 | 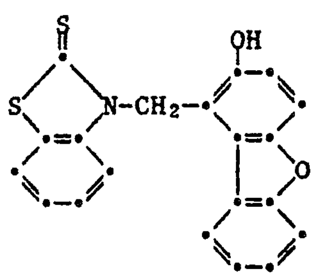 | | | |

Von den Verbindungen der Formel I ist die Verbindung der Formel

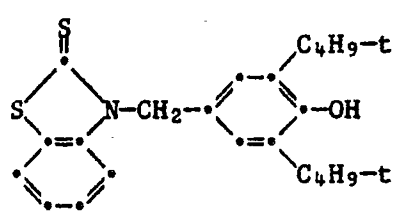

eine bekannte Verbindung. Gemäss SU-A-1,164.233 kann diese Verbindung als Metalldesaktivator in Polyolefinen verwendet werden.

Alle anderen Verbindungen der Formel I sind neue Verbindungen und sind als solche ebenfalls Gegenstand der vorliegenden Erfindung.

Die Herstellung dieser Verbindungen kann durch Erwärmen der entsprechenden S-substituierten Isomeren der Formel V geschehen:

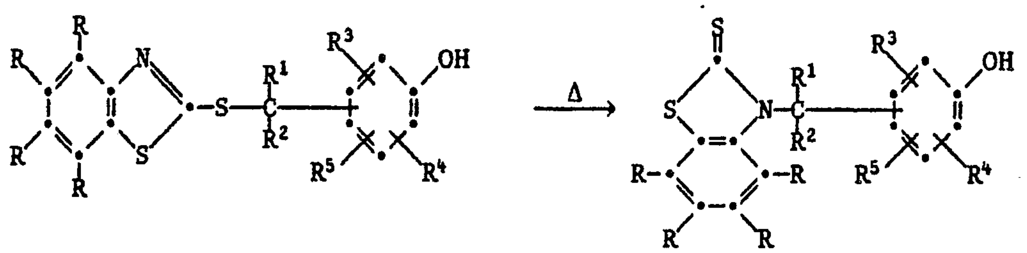

V I

Das Erwärmen kann mit oder ohne Lösungsmittel erfolgen. Als Lösungsmittel eignen sich z.B. aromatische Kohlenwasserstoffe, wie Toluol oder Xylol; Halogenkohlenwasserstoffe, wie Tetrachlorethylen oder Chlorbenzol; Alkanole, wie Isopropanol oder n-Butanol; Ester, Ketone, Dimethylformamid oder Dimethylsulfoxid. Polare Lösungsmittel, wie z.B. Dimethylformamid, beschleunigen die Reaktion. Die Umlagerung kann ferner durch Zusatz von basischen Katalysatoren beschleunigt werden. Beispiele für solche sind vor allem aliphatische, cycloaliphatische oder heterocyclische Amine. Wenn die phenolische OH-Gruppe in Paraposition zum Rest

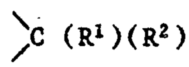

steht, sc verläuft die Umlagerung schneller als wenn sie in Orthoposition steht. Die zur Umlagerung erforderliche Temperatur hängt daher von der Position der OH-Gruppe und dem verwendeten Lösungsmittel und Katalysator ab. Vorzugsweise arbeitet man bei 70-250 °C, insbesondere bei 100-200 °C.

Die Ausgangsverbindungen der Formel V sind bekannte Verbindungen oder können in Analolgie zu den bekannten Verbindungen hergestellt werden. Ihre Herstellung kann durch Umsetzung der entsprechenden 2-Mercaptobenzthiazole VI mit einer Carbonylverbindung VII und einem Phenol VIII unter saurer Katalyse erfolgen nach dem Schema

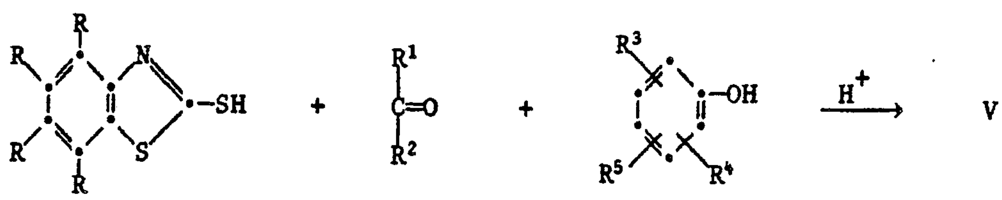

VI VII VIII

wie es z.B. im US-A-3,281.473 beschrieben ist. Alternativ kann man V aus VI auch durch Reaktion mit dem entsprechenden Benzylalkohol IX herstellen:

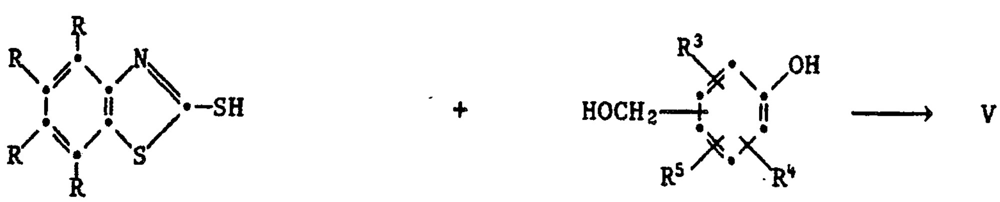

VI IX

wie es z.B. im US-A-3,215.641 beschrieben ist.

Eine zweite Herstellungsmöglichkeit für Verbindungen der Formel I ist die Reaktion von 2-Mercaptobenzthiazolen der Formel VI mit einem N-disubstituierten Aminomethylphenol, der Formel X:

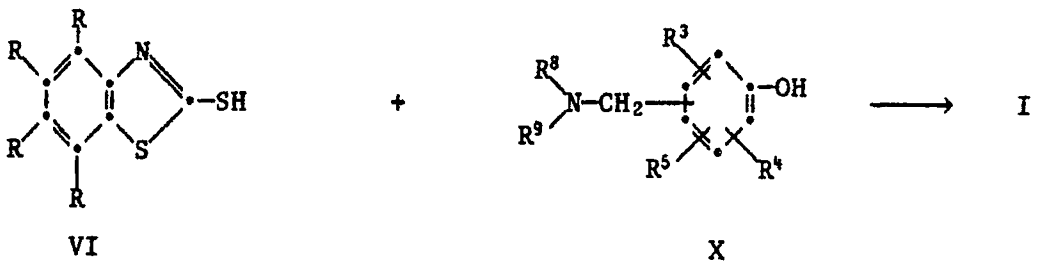

Darin bedeuten $R^8$ und $R^9$ unabhängif voneinander $C_1$-$C_{12}$-Alkyl, $C_5$-$C_8$-Cycloalkyl, Benzyl oder Phenyl. Diese Reaktion ist im SU-A-1,164.233 beschrieben. Sie wird vorzugsweise in einem polaren organischen Lösungsmittel ausgeführt. Beispiele hierfür sind niedrige Alkanole ($C_1$-$C_4$), Dimethylformamid oder Dimethylsulfoxid. Die Reaktion wird durch Erwärmen auf 50-200°C, vorzugsweise auf 70-150° C, ausgeführt.

Eine dritte Herstellungsmöglichkeit ist die Reaktion eines 2-Mercaptobenzthiazols der Formel VI mit einer Carbonylverbindung der Formel VII und einem Phenol der Formel VIII unter basischer Katalyse:

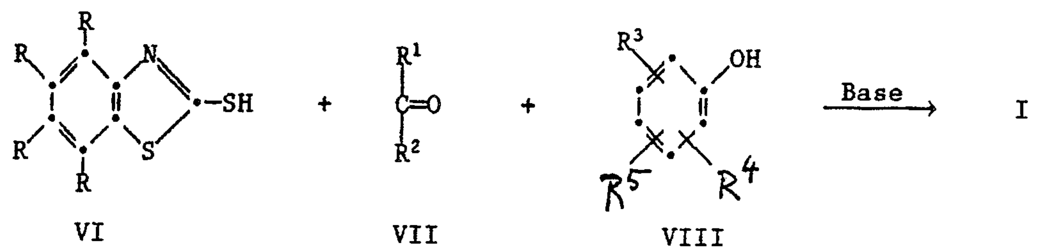


Während sich, wie vorhin beschrieben, unter saurer Katalyse die S-substituierten Isomeren der Formel V bilden, entstehen bei derselben Reaktion unter basischer Katalyse die N-substituierten Isomeren der Formel I.

Die Reaktion wird bevorzugt in einem polaren Lösungsmittel durch Erwärmen auf 50-150° C, vorzugsweise auf 70-120° C ausgeführt.

Als basische Katalysatoren kommen alle bekannten organischen oder anorganischen starken Basen in Betracht. Vorzugsweise verwendet man primäre, sekundäre oder tertiäre Amine, wie z.B. Isopropylamin, Butylamin, Cyclohexylamin, Dibutylamin, Dihexylamin, Di(isopropyl)-amin, Triethylamin, Tributylamin, Piperidin, Morpholin, Pyrrolidin oder Chinolin. Die Reaktion eignet sich vor allem gut bei Verwendung von Formaldehyd als Carbonylverbindung, wobei Produkte der Formel I mit $R^1 = R^2 = H$ entstehen. Der Formaldehyd kann z.B. als wässrige Lösung (Formalin) oder in Form von Paraformaldehyd verwendet werden oder man verwendet ein Reagens, das unter den Reaktionsbedingungen Formaldehyd bildet, wie z.B. Hexamethylentetramin.

Diese Reaktion eignet sich auch zur Herstellung von Verbindungen der Formel I, worin $R^3$ oder $R^4$ eine Gruppe der Formel II ist. In diesem Fall verwendet man ein Phenol der Formel VIII, worin $R^3$ oder $R^4$ Wasserstoff ist und setzt es mit mindestens zwei Aequivalenten Mercaptobenzthiazol der Formel VI und mindestens zwei Aequivalenten der Carbonylverbindung der Formel VII um:

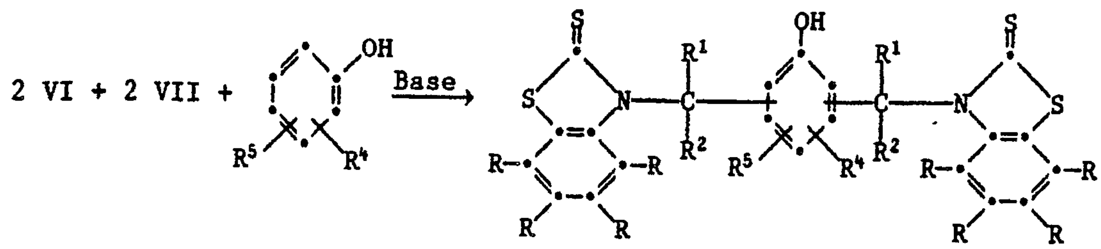


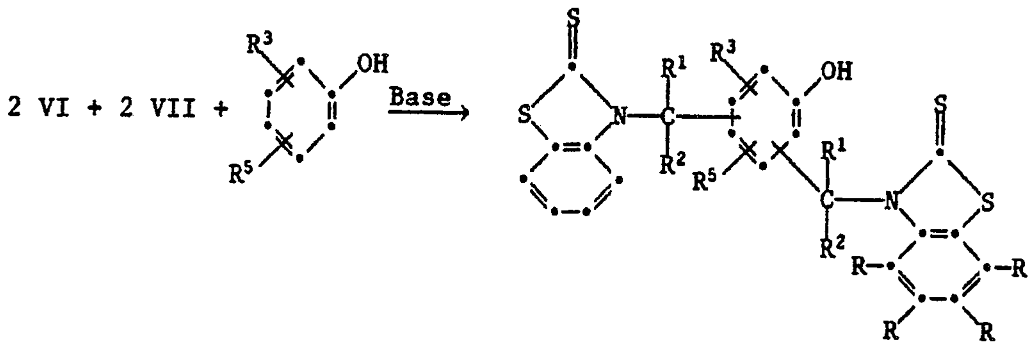


Die Verbindungen der Formel I sind als Korrosionsinhibitoren und als Antioxidantien wirksam. Als solche können sie allen flüssigen oder festen organischen Materialien zugesetzt werden. Bevorzugt verwendet man sie in Anstrichstoffen oder in Schmiermitteln.

Anstrichstoffe sind z.B. Lacke, Farben oder Firnisse. Sie enthalten stets ein filmbildendes Bindemittel neben anderen fakultativen Komponenten.

Beispiele für Anstrichstoffe sind solche auf Basis eines Epoxid-, Polyurethan-, Aminoplast-, Acryl-, Alkyd- oder Polyesterharzes sowie vom Gemischen solcher Harze. Weitere Beispiele geeigneter Bindemittel sind Vinylharze wie Polyvinylacetat, Polyvinylbutyral, Polyvinylchlorid und deren Copolymere, Celluloseester, chlorierte Kautschuke, Phenolharze, Styrol-Butadien-Copolymere und trocknende Oele.

Die Anstrichstoffe können lösungsmittelhaltig oder lösungsmittelfrei sein oder sie können wässrige Systeme (Dispersionen, Emulsionen, Lösungen) darstellen. Sie können pigmentiert oder unpigmentiert sein, sie können auch metallisiert sein. Ausser den erfindungsgemässen Korrosionsinhibitoren können sie sonstige in der Technologie der Anstrichstoffe übliche Zusätze enthalten, wie z.B. Füllstoffe, Verlaufhilfsmittel, Dispersierhilfsmittel, Thixotropiemittel, Haftverbesserer, Antioxidantien, Lichtschutzmittel oder Härtungskatalysatoren. Sie können auch andere bekannte Korrosionsschutzmittel enthalten, beispielsweise Korrosionsschutz-Pigmente, wie phosphat- oder borathaltige Pigmente oder Metalloxid-Pigmente, oder andere organische oder anorganische Korrosionsinhibitoren, z.B. Salze der Nitroisophthalsäure, Phosphorester, technische Amine oder substituierte Benztriazole.

Von Vorteil ist ferner der Zusatz von basischen Füllstoffen oder Pigmenten, die in bestimmten Bindemittelsystemen einen synergistischen Effekt auf die Korrosionsinhibierung bewirken. Beispiele für solche basische Füllstoffe und Pigmente sind Calcium- oder Magnesiumcarbonat, Zinkoxid, Zinkcarbonat, Zinkphosphat, Magnesiumoxid, Aluminiumoxid, Aluminiumphosphat oder Gemische davon. Beispiele für basische organische Pigmente sind solche auf Basis von Aminoanthrachinon.

Der Korrosionsinhibitor kann auch auf einem Trägerstoff aufgebracht werden. Hierfür eignen sich insbesondere pulverförmige Füllstoffe oder Pigmente. Diese Technik ist in der DE-A-3 122 907 näher beschrieben.

Die Korrosionsinhibitoren können den Anstrichstoff während dessen Herstellung zugesetzt werden, z.B. während der Pigmentverteilung durch Mahlen, oder man löst den Inhibitor in einem Lösungsmittel vor und rührt die Lösung in das Ueberzugsmittel ein. Man verwendet den Inhibitor in einer Menge von 0,1 bis 20 Gew.%, vorzugsweise 0,5 bis 5 Gew.%, bezogen auf den Feststoffgehalt des Anstrichstoffes.

Die Anstrichstoffe können nach den üblichen Verfahren auf das Substrat aufgebracht werden, z.B. durch Sprühen, Tauchen, Streichen oder durch Elektroabscheidung, insbesondere kathodische Tauchlackierung. Oft werden mehrere Schichten aufgetragen. Die Korrosionsinhibitoren werden in erster Linie der Grundschicht (Primer) zugegeben, da sie vor allem an der Grenze Metall-Anstrich wirken. Man kann aber die Inhibitoren auch zusätzlich der Deckschicht oder Zwischenschicht zugeben, wo sie als Depot zur Verfügung stehen. Je nachdem, ob das Bindemittel ein physikalisch trocknendes Harz oder ein hitze- oder strahlenhärtbares Harz ist, erfolgt die Härtung des Anstriches bei Raumtemperatur oder durch Erwärmen (Einbrennen) oder durch Bestrahlung.

Vorzugsweise ist der Anstrichstoff ein Grundanstrich (Primer) für metallische Substrate, wie z.B. Eisen, Stahl, Kupfer, Zink oder Aluminium. Der Anstrichstoff kann ein wässriges System sein, insbesondere ein kathodisch abscheidbarer Anstrichstoff (Kataphoreselack).

Zusätzlich zur antikorrosiven Wirkung haben die Verbindungen der Formel I den Vorteil, dass sie die Adhäsion Anstrich-Metall günstig beeinflussen und schliesslich, dass sie eine antioxidative und lichtschützende Wirkung auf den Anstrich ausüben und damit das Auskreiden von Pigmenten und Füllstoffen vermindern. Alle diese Eigenschaften tragen zu einer Verlängerung der Gebrauchsdauer des Anstriches bei.

Beispiele für Schmiermittel, denen man die erfindungsgemässen Korrosionsinhibitoren zusetzen kann, sind Schmieröle und Schmierfette. Die Schmieröle können Mineralöle oder synthetische Oele sein oder Gemische von beiden. Synthetische Oele sind beispielsweise solche auf Basis von Phosphorsäureestern, von Polyalkylenoxiden, von $\alpha$-Olefin-Polymerisaten, von Triestern des Trimethylolpropan oder Tetraestern des Pentaerythritol oder von aliphatischen Polyestern.

Die Schmiermittel können weitere Additive enthalten, wie z.B. Antioxidantien, Stockpunktserniedriger, Viskositätsindex-Verbes-serer, Metalldesaktivatoren, Dispergiermittel, Hochdruckadditive oder Verschleissschutzadditive. Sie können auch noch andere Korrosionsinhibitoren enthalten, wie z.B. organische Säuren und deren Ester, Metallsalze, Aminsalze oder Anhydride, heterocyclische Verbindungen, Phosphorsäurepartialester und deren Aminsalze oder Metallsalze von Sulfonsäuren.

Für die Verwendung der Verbindungen der Formel I in Schmiermitteln ist es von erheblicher Bedeutung, dass diese Verbindungen auch als Antioxidantien wirken, da auf diesem Sektor Mehrzweck-Additive besonders wertvoll sind.

Man verwendet die Verbindungen der Formel I in Schmiermitteln in einer Menge von 0,01 bis 5 Gew.%, insbesondere 0,2 bis 2 Gew.%.

Sowohl für Anstrichstoffe wie für Schmiermittel kann der Zusatz eines Gemisches von mehreren Verbindungen der Formel I von Bedeutung sein. Beispielsweise kann bei Einsatz bestimmter technischer Phenolgemische bei der Herstellung der Verbindungen der Formel I zwangsläufig ein Gemisch von Produkten der Formel I entstehen, das als solches verwendet werden kann. Es kann aber auch zur Herabsetzung des Schmelzpunktes von Vorteil sein, zwei oder mehr solcher Verbindungen zu mischen.

Herstellung und Verwendung von Verbindungen der Formel I sind in den nachfolgenden Beispielen näher beschrieben. Hierbei beziehen sich Teile und Prozente auf Gewichtsteile und Gewichtsprozente, sofern nicht anders angegeben. Die Temperatur ist in °C angegeben.

Beispiel 1: 2,0 g 2-(3,5-Di-tert.butyl-4-hydroxybenzylthio)-benzthiazol (hergestellt gemäss US-A-3,215.641, Beispiel 1) werden in 10 ml Dimethylformamid (DMF) gelöst und die Lösung 2,5 Stunden unter $N_2$ auf 150° erwärmt. Das Lösungsmittel wird dann im Vakuum abdestilliert und das gelbliche Rohprodukt aus Ethanol umkristallisiert. Man erhält 1,8 g 3-(3,5-Di-tert.butyl-4-hydroxybenzyl)-benzthiazol-2-thion von Smp. 148-150° (Verbindung Nr. 1).

Beispiel 2: Verfährt man wie im Beispiel 1 beschrieben unter Einsatz von 2-(3-tert.Butyl-2-hydroxy-5-methylbenzylthio)-benzthiazol, so erhält man 3-(3-tert.Butyl-2-hydroxy-5-methylbenzyl)-benzthiazol-2-thion, das bei 178-180° schmilzt (Verbindung Nr. 30).

Beispiel 3: Zu einer Suspension von 264,3 g 2,6-Diphenylphenol (1 Mol) in 1,5 1 95%igem Ethanol und 20 g DMF werden unter schnellem Rühren 135,2 g einer 40%igen wässrigen Lösung von Dimethylamin (1,2 Mol) zugegeben. Anschliessend tropft man 98,3 g einer 37%igen wässrigen Formaldehydlösung innerhalb 30 Min. bei Raumtemperatur zu. Die Suspension wird 70 Stunden bei Raumtemperatur gerührt und anschliessend filtriert. Der Filterrückstand wird mit kaltem 80%igem Ethanol gewaschen und aus 1,5 l Acetonitril umkristallisiert. Man erhält 262,9 g N,N-Dimethyl-3,5-diphenyl-4-hydroxybenzylamin, das bei 136-137° schmilzt.

30,3 g dieses Amines (0,1 Mol) und 16,7 g Mercaptobenzthiazhol (0,1 Mol) werden in 100 ml DMF gelöst und die Lösung 42 Stunden unter $N_2$ auf 110° erwärmt. Dann wird die Lösung im Vakuum eingedampft und das zurückbleibende Oel aus Ethanol kristallisiert. Man erhält 42,6 g 3-(3,5-Diphenyl-4-hydroxybenzyl)-benzthiazol-2-thion als gelbe Kristalle, die bei 145-146° schmelzen (Verbindung Nr. 3).

Beispiel 4: Analog Beispiel 3 erhält man aus N,N-Dimethyl-3,5-di-tert.butyl-4-hydroxybenzylamin das 3-(3,5-Di-tert.butyl-4-hydroxybenzyl)-benzthiazol-2-thion, das nach zweimaligem Umkristallisieren bei 152-154° schmilzt (Verbindung Nr. 1).

Beispiel 5: Analog Beispiel 3 erhält man aus N,N-Dimethyl-3-tert.butyl-4-hydroxy-5-methylbenzylamin und 2-Mercaptobenzthiazol das 3-(3-tert.Butyl-4-hydroxy-5-methylbenzyl)-benzthiazol-2-thion vom Smp. 153-155° (Verbindung Nr. 2).

Beispiel 6: 66,9 g 2-Mercaptobenzthiazol (0,4 Mol) und 82,5 g 2,6-Di-tert.butylphenol (0,4 Mol) werden in 100 ml DMF suspendiert. 43 g einer 35%igen wässrigen Formaldehydlösung (0,5 Mol) und 2,6 g Dibutylamin (0,02 Mol) werden zugegeben und die Dispersion 4 Stunden unter $N_2$ bei 90° gerührt. Das

Reaktionsgemisch wird im Vakuum eingedampft, das erhaltene Rohprodukt aus Ethylacetat/Hexan umkristallisiert. Man erhält 120 g 3-(3,5-Di-tert.butyl-4-hydroxybenzyl)-benzthiazol-2-thion als gelbes Pulver, das bei 148-151° schmilzt (Verbindung Nr. 1).

Beispiel 7: Analog erhält man aus 2-tert.Butyl-6-methylphenol, 2-Mercaptobenzthiazol und Formaldehyd unter Katalyse von Dibutylamin das 3-(3-tert.Butyl-4-hydroxy-5-methylbenzyl)-benzthiazol-2-thion, das nach zweimaliger Umkristallisation aus 70%igem wässrigem Ethanol bei 156-157° schmilzt (Verbindung Nr. 2).

Beispiel 8: Analog erhält man aus 0,2 Mol 2,6-Diisopropylphenol, 0,2 Mol 2-Mercaptobenzthiazol und 0,25 Mol Formaldehyd in 175 ml DMF das 3-(3,5-Diisopropyl-4-hydroxybenzyl)-benzthiazol-2-thion, das nach Umkristallisation aus Ethylacetat/Petrolether 60-80 bei 114-115° schmilzt (Verbindung Nr. 10).

Beispiel 9: Analog Beispiel 8 erhält man bei Verwendung von 2,6-Dimethylphenol das 3-(3,5-Dimethyl-4-hydroxybenzyl)-benzthiazol-2-thion vom Smp. 164-165° (Verbindung Nr. 4).

Beispiel 10: In Analogie zu Beispiel 6 werden die folgenden Verbindungen hergestellt:
3-(3,5-Di-sec-butyl-4-hydroxybenzyl)-benzthiazol-2-thion (Verbindung Nr. 11), Smp.;
3-(3,5-Di-cyclohexyl-4-hydroxybenzyl)-benzthiazol-2-thion (Verbindung Nr. 6), Smp. 184°;
3-(3-Cyclohexyl-5-tert.butyl-4-hydroxybenzyl)-benzthiazol-2-thion (Verbindung Nr. 23), Smp. 149°;
3-(3-Phenyl-5-methyl-4-hydroxybenzyl)-benzthiazol-2-thion (Verbindung Nr. 24), Smp. 147-149°;
3-(3-Methyl-5-cyclohexyl-4-hydroxybenzyl)-benzthiazol-2-thion (Verbindung Nr. 5), Smp. 148°;
3-(2,3,5-Trimethyl-4-hydroxybenzyl)-benzthiazol-2-thion (Verbundung Nr. 21), Smp. 227-228°;
5-Chlor-3-(3,5-di-tert.butyl-4-hydroxybenzyl)-benzthiazol-2-thion (Verbindung Nr. 25), Smp. 147-149°;
6-Ethoxy-3-(3,5-di-tert.butyl-4-hydroxybenzyl)-benzthiazol-2-thion (Verbindung Nr. 26), Smp. 184°;
5-Nitro-3-(2,5-di-tert.butyl-4-hydroxybenzyl)-benzthiazol-2-thion (Verbindung Nr. 22);
5-Trifluormethyl-3-(3,5-di-tert.butyl-4-hydroxybenzyl)-benzthiazol-2-thion (Verbindung Nr. 20), Smp. 161-162°;
3-(3-Methyl-5-allyl-4-hydroxybenzyl)-benzthiazol-2-thion (Verbindung Nr. 28), Smp. 104-106°.

Beispiel 11: 3-(3,5-Di-tert.butyl-2-hydroxybenzyl)-benzthiazol-2-thion (Verbindung 31) wird hergestellt durch 4-stündiges Erwärmen von 0,2 Mol 2-Mercaptobenzthiazol, 0,2 Mol 2,4-Di-tert.butylphenol und 0,2 Mol Paraformaldehyd in Gegenwart von 1 ml Dibutylamin auf 150° und Kristallisation des Rohproduktes aus Ethanol.

| | | | | |
|---|---|---|---|---|
| Analyse $C_{22}H_{27}N_4OS_4$ | ber. C = 68,75 % | H = 7,11 % | N = 3,66 % |
| | gef. C = 68,57 % | H = 7.01 % | N = 3,63 % |

In analoger Weise werden die folgenden Verbindungen hergestellt:
3-(3,5-Dimethyl-2-hydroxybenzyl)-benzthiazol-2-thion (Verbindung Nr. 36), Smp. 155-157°;
3-(3,5-Di-isopropyl-2-hydroxybenzyl)-benzthiazol-2-thion (Verbindung Nr. 37), Smp. 150-152°;
3-(3,5-Di-tert.amyl-2-hydroxybenzyl)-benzthiazol-2-thion (Verbindung Nr. 50), Smp. 155-158°;
3-(3-sec.Butyl-5-tert.amyl-2-hydroxybenzyl)-benzthiazol-2-thion (Verbindung Nr. 51), Smp. 104-107°;
3-(3,5-Di-tert.octyl-2-hydroxybenzyl)-benzthiazol-2-thion (Verbindung Nr. 32), Smp. 122°;
3-(4-tert.Butyl-2-hydroxybenzyl)-benzthiazol-2-thion (Verbindung Nr. 52), Smp. 121-126°;
3-(4-Pentadecyl-2-hyxdroxybenzyl)-benzthiazol-2-thion (Verbindung Nr. 53), Smp. 105-106;
3-(4-Pentadecenyl-2-hydroxybenzyl)-benzthiazol-2-thion (Verbindung Nr. 55), roter Sirup;
3-(3-Methyl-5-allyl-2-hydroxybenzyl)-benzthiazol-2-thion (Verbindung Nr. 54), Smp. 90-93°.

Beispiel 12: 16.7 g 2-Mercaptobenzthiazol, 3,0 g Paraformaldehyd, 15,4 g 2,6-Dimethoxyphenol und 1 g Dibutylamin werden 2 Std. unter Rühren auf 110° erwärmt. Nach Zugabe von 50 ml Ethanol wird das Reaktionsgemisch auf 10° gekühlt. Man erhält 27,3 g 3-(3,5-Dimethoxy-4-hydroxybenzyl)-benzthiazol-2-thion (Verbindung Nr. 8), die bei 141-142° schmilzt.

Analyse $C_{16}H_{15}NO_3S_2$ ber. S = 19,3 %; gef. S = 19,22 %.

Ersetzt man in dieser Prozedur das Dimethoxyphenol durch 16,3 g 2,6-Dichlorphenol, so erhält man 24,3 g 3-(3,5-Dichlor-4-hydroxybenzyl)-benzthiazol-2-thion (Verbindung Nr. 9), die bei 169-172° schmilzt.

Analyse $C_{14}H_9NOS_2Cl_2$ ber. Cl = 20,96 %; gef. Cl = 20,76 %.

Beispiel 13: Nach der Methode des Beispiels 12 werden 33,4 g Mercaptobenzthiazol, 6 g Paraformaldehyd, 31,8 g 8-Hydroxychinaldin und 2 ml Dibutylamin umgesetzt. Man erhält 64 g 3-(2-Methyl-8-hydroxychinolin-7-ylmethyl)-benzthiazol-2-thion (Verbindung Nr. 60), die bei 211-215° schmilzt.

Ersetzt man in dieser Prozedur das Hydroxychinaldin durch 36,8 g 2-Hydroxydibenzofuran, so erhält man 41 g 3-(2-Hydroxydibenzofuran-1-ylmethyl)-benzthiazol-2-thion (Verbindung Nr. 61), das bei 174-180° schmilzt.

Beispiel 14: 6,8 g 2-(5-Nitro-2-hydroxybenzyl)-benzthiazol werden in 30 ml Dimethylformamid unter Zusatz von 0,2 g Dibutylamin 12 Stunden zum Rückfluss erhitzt. Nach Abdestillieren des Lösungsmittels wird das Rohprodukt durch Säulenchromatographie gereinigt. Man erhält 3 g
3-(5-Nitro-2-hydroxybenzyl)-benzthiazol-2-thion (Verbindung Nr. 40), die bei 231° schmilzt.

Beispiel 15: Ein Alkydharz-Lack wird nach folgender Rezeptur bereitet:

40 Teile Alphthalat® AM 380 (60%ige Xylol-Lösung) Alkydharz der Fa. Reichhold Albert Chemie AG
10 Teile Eisenoxidrot 225 der Fa. Bayer AG
13,6 Teile Talkum (mikronisiert)
13 Teile mikronisiertes Calciumcarbonat (Millicarb®, Plüss-Staufer AG)
0,3 Teile Hautverhütungsmittel Luaktin® (BASF)
0,6 Teile 8%ige Cobaltnaphthenat-Lösung
22,5 Teile Xylol/Ethylglykol-Gemisch 6:40.

Der Lack wird mit Glasperlen gemahlen bis zu einer Pigement- und Füllstoffkorngrösse von 10-15 µm. Vor dem Mahlen werden die in der folgenden Tabelle angegebenen Korrosionsinhibitoren zugegeben.

Der Lack wird auf sandgestrahlte Stahlbleche von 7 x 13 cm aufgespritzt in einer Schichtdicke, die nach dem Trocknen ca. 50 µm beträgt. Nach 7 Tragen Trocknung bei Raumtemperatur werden die Proben 60 Min. bei 60° nachgehärtet.

In die gehärtete Lackoberfläche werden zwei kreuzförmige Schnitte von 4 cm Länge bis zum Metall eingeschnitten mit Hilfe eines Bonder-Kreuzschnittgerätes. Zum Schutze der Kanten wird auf diese ein Kantenschutzmittel (Icosit® 255) aufgebracht.

Die Proben werden nunmehr einem Salzsprühtest gemäss ASTM B 117 mit einer Dauer von 600 Stunden unterworfen. Nach jeweils 200 Stunden Bewitterung wird der Zustand des Anstriches beurteilt, und zwar der Blasengrad (gemäss DN 53 209) am Kreuzschnitt und auf der lackierten Fläche sowie der Rostgrad (gemäss DIN 53 210) auf der ganzen Fläche.

Nach Ende des Tests wird der Anstrich durch Behandlung mit konzentrierter Natronlauge entfernt und die Korrosion des Metalls am Kreuzschnitt (gemäss DIN 53 167) sowie über die restliche Fläche beurteilt. Die Beurteilung erfolgt jeweils in einer 6-Stufen-Skala. Die Summe der Bewertung des Anstriches und der Bewertung der Metalloberfläche ergibt den Korrosionsschutzwert KS. Je höher dieser ist, desto wirkungsvoller ist der getestete Inhibitor.

Tabelle 1: Ergebnisse des Salzsprühtestes

| Korrosions-inhibitor | Zusatz-menge*) | Beurteilung Anstrich | Beurteilung Metall | KS |
|---|---|---|---|---|
| Keiner | - | 2,2 | 1,7 | 3,9 |
| Verbindung Nr. 1 | 2 % | 5,0 | 5,1 | 10,1 |
| Verbindung Nr. 2 | 2 % | 3,9 | 4,2 | 8,1 |
| | 4 % | 4,4 | 4,6 | 9,0 |
| Verbindung Nr. 3 | 2 % | 4,9 | 3,9 | 8,8 |
| | 4 % | 5,1 | 4,7 | 9,8 |
| Verbindung Nr. 4 | 4 % | 2,9 | 2,0 | 4,9 |
| Verbindung Nr. 6 | 4 % | 3,5 | 2,4 | 5,9 |
| Verbindung Nr. 11 | 4 % | 3,3 | 2,3 | 5,6 |
| Verbindung Nr. 20 | 2 % | 4,4 | 4,5 | 8,9 |
| Verbindung Nr. 21 | 2 % | 4,8 | 5,2 | 10,0 |
| Verbindung Nr. 22 | 2 % | 4,0 | 5,2 | 9,2 |
| Verbindung Nr. 23 | 2 % | 4,4 | 4,5 | 8,9 |
| Verbindung Nr. 25 | 2 % | 4,8 | 2,3 | 7,1 |
| Verbindung Nr. 26 | 2 % | 2,9 | 3,3 | 6,2 |
| Verbindung Nr. 27 | 2 % | 3,8 | 5,1 | 8,9 |
| Verbindung Nr. 29 | 2 % | 3,7 | 3,0 | 6,7 |
| Verbindung Nr. 32 | 4 % | 3,9 | 2,0 | 5,9 |
| Verbindung Nr. 38 | 2 % | 4,3 | 4,5 | 8,8 |
| Verbindung Nr. 43 | 2 % | 3,6 | 5,0 | 9,6 |
| Verbindung Nr. 49 | 2 % | 4,4 | 4,5 | 8,9 |
| Verbindung Nr. 50 | 4 % | 3,0 | 1,9 | 4,9 |
| Verbindung Nr. 51 | 2 % | 3,9 | 3,1 | 7,0 |
| Verbindung Nr. 52 | 2 % | 4,5 | 4,5 | 9,0 |
| Verbindung Nr. 53 | 2 % | 5,8 | 5,8 | 11,6 |
| Verbindung Nr. 55 | 2 % | 4,0 | 4,9 | 8,9 |
| Verbindung Nr. 56 | 4 % | 3,3 | 2,3 | 5,6 |
| Verbindung Nr. 58 | 4 % | 2,0 | 3,2 | 5,2 |
| Verbindung Nr. 59 | 4 % | 3,7 | 3,1 | 6,8 |

*) bezogen auf den Festkörpergehalt des Lackes

Beispiel 16: Es werden Stahlbleche wie in Beispiel 15 beschrieben mit einem Alkydharzlack beschichtet. Die Proben werden mit einem Kreuzschnitt versehen und einer natürlichen Bewitterung in North-Carolina nahe der Meeresküste ausgesetzt. Nach 15 Monaten wird die Breite der Rostzone entlang den Schnittlinien gemessen gemäss Methode ASTM D 1654-79a. Die Ergebnisse sind in Tabelle 2 aufgeführt.

Tabelle 2:

| Korrosions-inhibitor | Breite der Rostzone (mm) |
|---|---|
| Keiner | 2 - 3 |
| 2 % Verbindung Nr. 1 | 0,5 - 1 |
| 2 % Verbindung Nr. 2 | 0,5 - 1 |
| 2 % Verbindung Nr. 3 | 0 - 0,5 |

Beispiel 17: Ein schwarz pigmentierter Kataphorese-Primer, hergestellt nach US-A-4 148 772/Example 1, wird auf zinkphosphatierten Stahlblechen kathodisch in einer Stärke von 20 µm abgeschieden. Die Lackierung wird 20 Minuten bei 180° eingebrannt. Ein Teil der Lackproben enthält 2 % der Verbindung Nr. 1. Die Proben werden dem Salzsprühtest nach ASTM B 117 unterworfen. Die Korrosion wird wie in Beispiel 15 beurteilt. Die Resultate sind in Tabelle 3 aufgeführt.

Tabelle 3:

| Korrosions-inhibitor | Beurteilung Anstrich | Beurteilung Metall | KS |
|---|---|---|---|
| Keiner | 2,2 | 1,3 | 3,5 |
| 2 % Verbindung Nr. 1 | 3,4 | 5,0 | 8,4 |

Beispiel 18: Ein Primer auf Basis Polyvinylbutyral/Epoxidharz wurde durch Mischen der folgenden Komponenten bereitet:
16 Teile eines aromatischen Epoxidharzes (Beckopox® EP 301) 50 %ige Lösung
40 Teile Polyvinylbutyral Mowital® B 30 HH - 20 %ige Lösung
8 Teile Talkum
12 Teile Eisenoxidrot 225 <Bayer AG)
2 Teile Epoxidharzhärter Beckopox EH 614
2 Teile Bariumsulfat
10 Teile Xylol
5 Teile Butanol
5 Teile Propylenglykol-monobutylether.

Ein Teil des Lackes wird mit 2 %, bezogen auf Festkörper, der Verbindung Nr. 1 versetzt. Der Primer wird in einer Schichtdicke von 40 m auf entfettete Stahlbleche aufgespritzt. Nach 7 Tagen Lufttrocknung werden die Proben dem Salzsprühtest nach ASTH B 117 unterworfen und die Korrosion wie in Beispiel 15 beurteilt. Die Werte sind in Tabelle 4 aufgeführt.

Tabelle 4:

| Korrosions-inhibitor | Beurteilung Anstrich | Beurteilung Metall | KS |
|---|---|---|---|
| Keiner | 2,2 | 3,1 | 5,3 |
| 2 % Verbindung Nr. 1 | 2,5 | 4,6 | 7,1 |

Beispiel 19: Es werden schwarz lackierte Proben hergestellt wie in Beispiel 17 beschrieben. Die gehärteten Proben werden in einem Xenon-Weatherometer 200 Std. bewittert. Der 60−-Glanz der Lackoberfläche wird mit einem Reflexionsphotometer gemessen. Ohne Zusatz eines Stabilisators ist ein rascher Abfall des Glanzes zu beobachten, verursacht durch den Photoabbau des Bindemittels und Auskreiden des Pigmentes.

**Tabelle 5:**

| **Stabilisator** | **% Glanzerhaltung nach 100 h** | **nach 200 h** | **WOM** |
|---|---|---|---|
| **Keiner** | **62** | **5** | |
| **2 % Verbindung Nr. 1** | **86** | **55** | |

Dieselben Proben werden einer natürlichen Bewitterung in Florida (45° Süd) unterworfen. Bereits nach 1 Monat zeigte sich ein deutlicher Unterschied zwischen den stabilisierten und den unstabilisierten Proben hinsichtlich Glanzerhaltung

**Tabelle 6:**

| **Stabilisator** | **% Glanzerhaltung nach einem Monat Florida** |
|---|---|
| **Keiner** | **32** |
| **2 % Verbindung Nr. 1** | **74** |

Aus beiden Tests zeigt sich, dass die Verbindungen der Formel I auch eine Lichtschutzwirkung besitzen.

Beispiel 20: Ein handelsüblicher grau pigmentierter Kataphorese-Primer wird an Stahlblechen kathodisch in einer Schichtdicke von 20 $\mu$m abgeschieden und bei 170° 30 Minuten eingebrannt. Ueber den Primer wird ein Klarlack auf Basis eines Zweikomponenten-Polyurethans in einer Schichtdicke von 40 $\mu$m aufgespritzt. Der Decklack wird 30 Minuten bei 120° gehärtet. Dann werden die Proben 1200 Stunden in einem Weatherometer bewittert. Die dabei auftretende Vergilbung wird durch Messung des Farbtonabstandes $\Delta E$ vom ursprünglichen Farbton (mittels eines Macbeth Colorimeters) bestimmt. Der Stabilisator wird dem Primer zugegeben. Die Resultate sind in Tabelle 7 aufgeführt.

Tabelle 7:

| Stabilisator | ΔE nach 400 h | 800 h | 1200 h WOM |
|---|---|---|---|
| Keiner | 6,8 | 8,0 | 8,9 |
| 2 % Verbindung Nr. 1 | 2,4 | 3,6 | 4,7 |

Beispiel 21: Der in Beispiel 17 beschriebene Kataphorese-Primer wird auf zinkphosphatierte Stahlbleche in einer Schichtdicke von 30 µm kathodisch abgeschieden und 20 Minuten bei 180° eingebrannt. Einem Teil der Proben werden 2% der Verbindung Nr. 1, bezogen auf das Lackharz des Primers, zugesetzt. Auf den gehärteten Primer wird ein weisser Decklack auf Basis eines Zweikomponenten-Polyurethans aufgetragen, der mit $TiO_2$ pigmentiert ist. Die Proben werden in einem Trockenofen mit Luftzirkulation 24 Std. auf 150° erwärmt. In bestimmten Zeitabständen wird der Yellowness-Index (ASTM D 1925-70) der Proben gemessen unter Benutzung eines Bariumsulfat-Standards. Die Ergebnisse sind in Tabelle 8 aufgeführt.

Tabelle 8:

| Stabilisator | Yellowness-Index nach 5 h | 9 h | 24 h |
|---|---|---|---|
| Keiner | 2,2 | 3,4 | 8,2 |
| 2 % Verbindung Nr. 1 | 1,1 | 2,0 | 5,2 |

Diese Ergebnisse zeigen, dass die Verbindungen der Formel I eine deutliche antioxidative Wirkung besitzen.

Beispiel 22: Dieses Beispiel zeigt die antioxidative Wirkung in einem Schmieröl. Der verwendete Test ist eine Modifikation des "Rotary Bomb Oxidation Test for Mineral Oils" (ASTM D 2272). Er ist beschrieben von C.S. Ku und S.M. Hsu in Lubrication Engineering, Vol. 40 (2), 75-83 (1984). Das verwendete Oel ist ein Motorenöl auf Basis Mineralöl, das 0,75 % Zink-dithiophosphat enthält. Dem Oel werden ausser dem zu prüfenden Stabilisator 2 % Wasser, 4 % einer oxidierten nitrierten Petroleumfraktion und 4 % eines flüssigen Metallnaphthenates zugesetzt. Diese Zusätze werden unter der Bezeichnung "Standard Reference Material No. 1817" vom National Bureau of Standards mit einem Analysen-Zertifikat geliefert.

In Tabelle 9 wird die Zeit angegeben bis zu einem deutliche Druckabfall. Lange Zeiten entsprechen einer guten Stabilisatorwirksamkeit.

Tabelle 9:

| Stabilisator | Minuten bis deutlichen Druckabfall |
|---|---|
| Keiner | 86 |
| 0,5 % Verbindung Nr. 31 | 134 |

**Ansprüche**

1. Zusammensetzung aus einem Anstrichstoff oder einem Schmiermittel, enthaltend mindestens eine Verbindung der Formel I

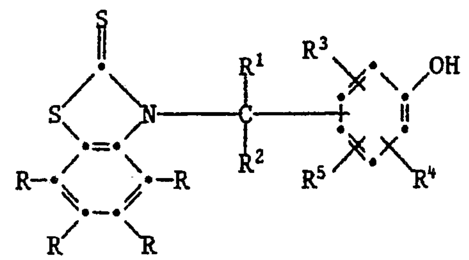

I

worin jedes R unabhängig voneinander Wasserstoff, $C_1$-$C_{12}$-Alkyl, $C_1$-$C_4$-Halogenalkyl, $C_1$-$C_{12}$-Alkoxy, $C_1$-$C_{12}$-Alkylthio, Phenylthio, Benzylthio, $C_1$-$C_{12}$-Alkylsulfonyl, Phenyl, $C_7$-$C_{15}$-Alkylphenyl, $C_7$-$C_{10}$-Phenylalkyl, $C_5$-$C_8$-Cycloalkyl, Halogen, $-NO_2$, -CN, -COOH -COO($C_1$-$C_4$-Alkyl), -OH, $-NH_2$, $-NHR^6$, $-N(R^6)_2$, $-CONH_2$, $-CONHR^6$ oder $-CON(R^6)_2$ bedeutet,
$R^1$ Wasserstoff, $C_1$-$C_{12}$-Alkyl, Phenyl, durch Halogen, $C_1$-$C_4$-Alkyl, $C_1$-$C_4$-Alkoxy oder $-NO_2$ substituiertes Phenyl, Pyridyl, Thienyl oder Furyl bedeutet,
$R^2$ Wasserstoff oder $C_1$-$C_4$-Alkyl bedeutet,
$R^3$ und $R^4$ unabhängig voneinander Wasserstoff, Halogen, $C_1$-$C_4$-Alkoxy, Cyano, Nitro, $C_1$-$C_{20}$-Alkyl, $-(CH_2)_m$-$COOR^7$, $-(CH_2)_m$-$CONHR^6$, $-(CH_2)_m$-$CON(R^6)_2$, $C_3$-$C_{20}$-Alkenyl, $C_7$-$C_{10}$-Phenylalkyl, Phenyl, Cyclohexyl, Cyclopentyl oder eine Gruppe der Formel II

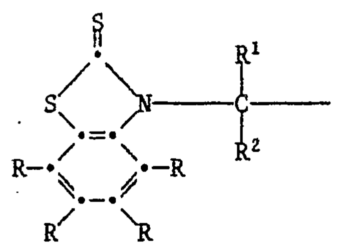

II

bedeuten,
$R^5$ Wasserstoff, $C_1$-$C_{20}$-Alkyl, $C_3$-$C_{20}$-Alkenyl oder Hydroxy bedeutet, oder $R^3$ und $R^5$ zusammen oder $R^4$ und $R^5$ zusammen einen Ring bilden, der am Phenolring anelliert ist und der ein carbocyclischer oder heterocyclischer Ring sein kann, welcher als Heteroatome Sauerstoff, Stickstoff oder Schwefel enthalten kann und welcher durch $C_1$-$C_4$-Alkyl, $C_1$-$C_4$-Alkoxy oder Halogen substituiert sein kann, $R^6$ $C_1$-$C_{12}$-Alkyl, durch ein oder mehrere O-Atome unterbrochenes $C_3$-$C_{12}$-Alkyl, $C_5$-$C_8$-Cycloalkyl, Benzyl, Phenyl oder durch Halogen, $C_1$-$C_4$-Alkyl, $C_1$-$C_4$-Alkoxy oder Nitro substituiertes Phenyl bedeutet oder $-N(R^6)_2$ eine Pyrrolidino-, Piperidino- oder Morpholinogruppe bedeutet,
$R^7$ Wasserstoff oder $C_1$-$C_{20}$-Alkyl bedeutet, das durch Halogen oder Hydroxyl substituiert sein kann, oder $C_3$-$C_{20}$-Alkyl bedeutet, das durch ein oder mehrere Sauerstoffatome unterbrochen ist und durch Hydroxyl substituiert sein kann, und m 0, 1 oder 2 bedeutet.

2. Zusammensetzung gemäss Anspruch 1, dadurch gekennzeichnet, dass in Formel I und II ein R Wasserstoff, $C_1$-$C_4$-Alkyl, $C_1$-$C_4$-Alkoxy, Halogen, Trifluormethyl oder Nitro bedeutet und die anderen drei R Wasserstoff sind.

3. Zusammensetzung gemäss Anspruch 1, dadurch gekennzeichnet, dass in Formel I $R^1$ Wasserstoff, $C_1$-$C_8$-Alkyl, Phenyl oder Furyl bedeutet und $R^2$ Wasserstoff ist.

4. Zusammensetzung gemäss Anspruch 1, dadurch gekennzeichnet, dass in Formel I $R^1$ und $R^2$ Wasserstoff sind.

5. Zusammensetzung gemäss Anspruch 1, dadurch gekennzeichnet, dass in Formel I $R^3$ und $R^4$ unabhängig voneinander Wasserstoff, $C_1$-$C_8$-Alkyl, Allyl, $C_7$-$C_{10}$-Phenylalkyl, $C_1$-$C_4$-Alkoxy, Halogen,

Phenyl, Cyclohexyl oder eine Gruppe $-CH_2CH_2COOR^7$ bedeutet, $R^5$ Wasserstoff, $C_1$-$C_{18}$-Alkyl, $C_3$-$C_{18}$-Alkenyl oder OH bedeutet und $R^7$ $C_1$-$C_{18}$-Alkyl ist oder $C_3$-$C_{20}$-Alkyl, das durch ein oder mehrere Sauerstoffatome unterbrochen ist.

6. Zusammensetzung gemäss Anspruch 1, enthaltend eine Verbindung der Formel III,

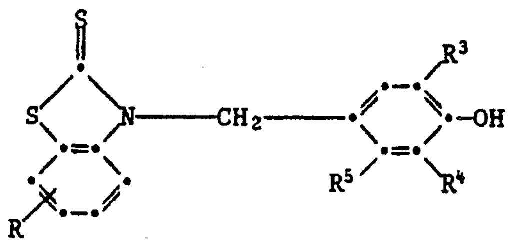

III

worin R Wasserstoff, $C_1$-$C_4$-Alkyl, $C_1$-$C_4$-Alkoxy, Chlor, Trifluormethyl oder Nitro ist, $R^3$ und $R^4$ unabhängig voneinander Wasserstoff, $C_1$-$C_8$-Alkyl, Allyl, Chlor, Methoxy, $C_7$-$C_{10}$-Phenylalkyl, Phenyl oder Cyclohexyl sind und $R^5$ Wasserstoff, Methyl oder OH ist.

7. Zusammensetzung gemäss Anspruch 1, enthaltend eine Verbindung der Formel IV,

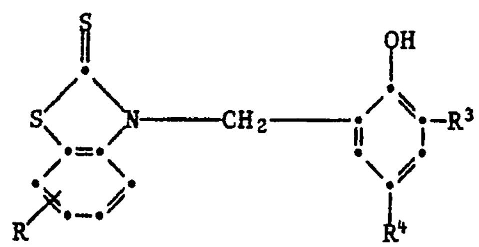

IV

worin R Wasserstoff, $C_1$-$C_4$-Alkyl, $C_1$-$C_4$-Alkoxy, Chlor, Trifluormethyl oder Nitro ist, $R^3$ und $R^4$ unabhängig voneinander Wasserstoff, $C_1$-$C_{18}$-Alkyl, $C_7$-$C_{10}$-Phenylalkyl, Phenyl, Cyclohexyl oder eine Gruppe der Formel IIa sind

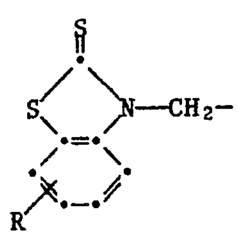

IIa

oder $R^3$ und $R^4$ zusammen einen anellierten Benzolring, Pyridinring oder Benzofuranring bilden.

8. Zusammensetzung gemäss Anspruch 1, welche ein Primer für metallische Substrate ist.

9. Zusammensetzung gemäss Anspruch 8, welche ein Primer auf Eisen, Stahl, Kupfer, Zink oder Aluminium ist.

10. Zusammensetzung gemäss Anspruch 1, welche ein wässriger Anstrichstoff ist.

11. Zusammensetzung gemäss Anspruch 10, welche ein kathodisch abscheidbarer Anstrichstoff ist.

12. Zusammensetzung gemäss Anspruch 1, enthaltend 0,5 bis 5 Gew.-%, bezogen auf den Feststoffgehalt des Anstrichstoffes, mindestens einer Verbindung der Formel I wie in Anspruch 1 definiert.

13. Anstrichstoff gemäss Anspruch 1 auf der Basis eines Epoxyharzes, eines Polyurethanes eines Aminoplastes oder eines Acryl-, Alkyd-oder Polyesterharzes oder einer Mischung solcher Harze.

**14.** Anstrichstoff gemäss Anspruch 1 auf der Basis eines Vinylpolymeren, eines Celluloseesters, eines Chlorkautschuks, eines Phenolharzes, eines Styrol-Butadien-Copolymeren oder eines trocknenden Oeles.

**15.** Zusammensetzung gemäss Anspruch 1, welche ein Schmiermittel ist.

**16.** Schmiermittelzusammensetzung gemäss Anspruch 1, enthaltend 0,2 bis 2 Gew.-% einer Verbindung der Formel I wie in Anspruch 1 definiert.

**17.** Schmiermittelzusammensetzung gemäss Anspruch 15, enthaltend eine Verbindung der Formel IV wie in Anspruch 7 definiert.

**18.** Verbindung der Formel I,

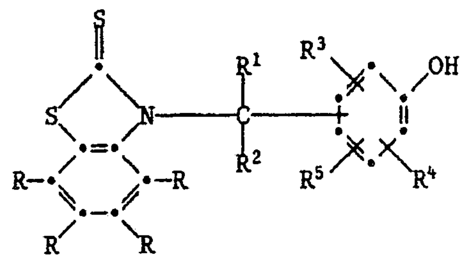

I

worin jedes R unabhängig voneinander Wasserstoff, $C_1$-$C_{12}$-Alkyl, $C_1$-$C_4$-Halogenalkyl, $C_1$-$C_{12}$-Alkoxy, $C_1$-$C_{12}$-Alkylthio, Phenylthio, Benzylthio, $C_1$-$C_{12}$-Alkylsulfonyl, Phenyl, $C_7$-$C_{15}$-Alkylphenyl, $C_7$-$C_{10}$-Phenylalkyl, $C_5$-$C_8$-Cycloalkyl, Halogen, $-NO_2$, -CN, -COOH, -COO($C_1$-$C_4$-Alkyl), -OH, $-NH_2$, $-NHR^6$, $-N(R^6)_2$, $-CONH_2$, $-CONHR^6$ oder $-CON(R^6)_2$ bedeutet, $R^1$ Wasserstoff, $C_1$-$C_{12}$-Alkyl, Phenyl, durch Halogen, $C_1$-$C_4$-Alkyl, $C_1$-$C_4$-Alkoxy oder $-NO_2$ substituiertes Phenyl, Pyridyl, Thienyl oder Furyl bedeutet, $R^2$ Wasserstoff oder $C_1$-$C_4$-Alkyl bedeutet, $R^3$ und $R^4$ unabhängig voneinander Wasserstoff, Halogen, $C_1$-$C_4$-Alkoxy, Cyano, Nitro, $C_1$-$C_{20}$-Alkyl, $-(CH_2)_m-COOR^7$, $-(CH_2)_m-CONHR^6$, $-(CH_2)_m-CON(R^6)_2$, $C_3$-$C_{20}$-Alkenyl, $C_7$-$C_{10}$-Phenylalkyl, Phenyl, Cyclohexyl, Cyclopentyl oder eine Gruppe der Formel II

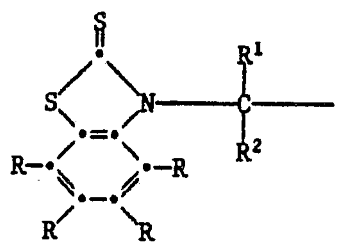

II

bedeuten,
$R^5$ Wasserstoff, $C_1$-$C_{20}$-Alkyl, $C_3$-$C_{20}$-Alkenyl oder Hydroxy bedeutet, oder $R^3$ und $R^5$ zusammen oder $R^4$ und $R^5$ zusammen einen Ring bilden, der am Phenolring anelliert ist und der ein carbocyclischer oder heterocyclischer Ring sein kann, welcher als Heteroatome Sauerstoff, Stickstoff oder Schwefel enthalten kann und welcher durch $C_1$-$C_4$-Alkyl, $C_1$-$C_4$-Alkoxy oder Halogen substituiert sein kann, $R^6$ $C_1$-$C_{12}$-Alkyl, durch ein oder mehrere O-Atome unterbrochenes $C_3$-$C_{12}$-Alkyl, $C_5$-$C_8$-Cycloalkyl, Benzyl, Phenyl oder durch Halogen, $C_1$-$C_4$-Alkyl, $C_1$-$C_4$-Alkoxy oder Nitro substituiertes Phenyl bedeutet oder $-N(R^6)_2$ eine Pyrrolidino-, Piperidino- oder Morpholinogruppe bedeutet, $R^7$ Wasserstoff oder $C_1$-$C_{20}$-Alkyl bedeutet, das durch Halogen oder Hydroxyl substituiert sein kann, oder $C_3$-$C_{20}$-Alkyl bedeutet, das durch ein oder mehrere Sauerstoffatome unterbrochen ist und durch Hydroxyl substituiert sein kann, und m 0, 1 oder 2 bedeutet mit der Massgabe, dass, wenn R, $R^1$ und $R^2$ Wasserstoff sind und $R^3$ und $R^4$ in Orthoposition zur Hydroxylgruppe stehende tert.Butylgruppen sind, $R^5$ nicht Wasserstoff ist.

**19.** Verfahren zur Herstellung von Verbindungen der Formel I in Anspruch 1, dadurch gekennzeichnet, dass man eine Verbindung der Formel V

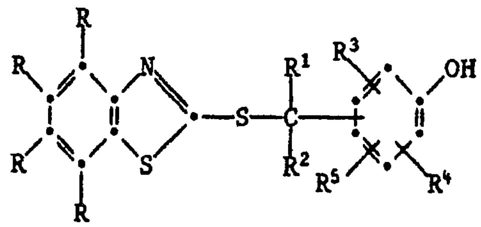

V

auf eine Temperatur von 70-250°, vorzugsweise 100-200° erhitzt.

**20.** Verfahren gemäss Anspruch 19, dadurch gekennzeichnet, dass man das Erhitzen in einem polaren Lösungsmittel ausführt.

**21.** Verfahren gemäss Anspruch 19, dadurch gekennzeichnet, dass man das Erhitzen in Gegenwart eines basischen Katalysators ausführt.

**22.** Verfahren zur Herstellung von Verbindungen der Formel I in Anspruch 1, dadurch gekennzeichnet, dass man ein 2-Mercaptobenzthiazol der Formel VI

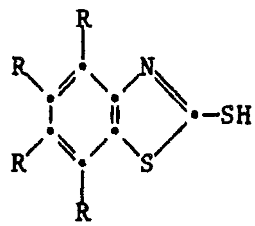

VI

und eine Carbonylverbindung

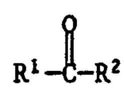

mit einem Phenol der Formel VIII

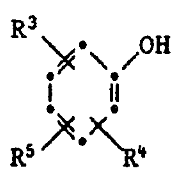

VIII

in Gegenwart eines basischen Katalysators umsetzt.

**23.** Verfahren gemäss Anspruch 22, dadurch gekennzeichnet, dass man die Reaktion in einem polaren Lösungsmittel ausführt.

Patentansprüche für folgende Vertragsstaaten : AT, ES

**1.** Zusammensetzung aus einem Anstrichstoff oder einem Schmiermittel, enthaltend mindestens eine Verbindung der Formel I

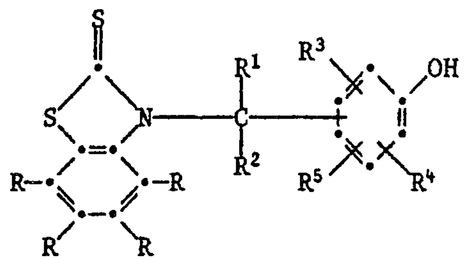

I

worin jedes R unabhängig voneinander Wasserstoff, $C_1$-$C_{12}$-Alkyl, $C_1$-$C_4$-Halogenalkyl, $C_1$-$C_{12}$-Alkoxy, $C_1$-$C_{12}$-Alkylthio, Phenylthio, Benzylthio, $C_1$-$C_{12}$-Alkylsulfonyl, Phenyl, $C_7$-$C_{15}$-Alkylphenyl, $C_7$-$C_{10}$-Phenylalkyl, $C_5$-$C_8$-Cycloalkyl, Halogen, $-NO_2$, -CN, -COOH, -COO($C_1$-$C_4$-Alkyl), -OH, $-NH_2$, $-NHR^6$, $-N(R^6)_2$, $-CONH_2$, $-CONHR^6$ oder $-CON(R^6)_2$ bedeutet,
$R^1$ Wasserstoff, $C_1$-$C_{12}$-Alkyl, Phenyl, durch Halogen, $C_1$-$C_4$-Alkyl, $C_1$-$C_4$-Alkoxy oder $-NO_2$ substituiertes Phenyl, Pyridyl, Thienyl oder Furyl bedeutet,
$R^2$ Wasserstoff oder $C_1$-$C_4$-Alkyl bedeutet,
$R^3$ und $R^4$ unabhängig voneinander Wasserstoff, Halogen, $C_1$-$C_4$-Alkoxy, Cyano, Nitro $C_1$-$C_{20}$-Alkyl, --$(CH_2)_m$-$COOR^7$, $-(CH_2)_m$-$CONHR^6$, $-(CH_2)_m$-$CON(R^6)_2$, $C_3$-$C_{20}$-Alkenyl, $C_7$-$C_{10}$-Phenylalkyl, Phenyl, Cyclohexyl, Cyclopentyl oder eine Gruppe der Formel II

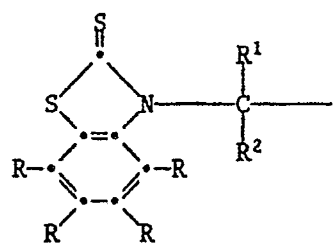

II

bedeuten,
$R^5$ Wasserstoff, $C_1$-$C_{20}$-Alkyl, $C_3$-$C_{20}$-Alkenyl oder Hydroxy bedeutet, oder $R^3$ und $R^5$ zusammen oder $R^4$ und $R^5$ zusammen einen Ring bilden, der am Phenolring annelliert ist und der ein carbocyclischer oder heterocyclischer Ring sein kann, welcher als Heteroatome Sauerstoff, Stickstoff oder Schwefel enthalten kann und welcher durch $C_1$-$C_4$-Alkyl, $C_1$-$C_4$-Alkoxy oder Halogen substituiert sein kann, $R^6$ $C_1$-$C_{12}$-Alkyl durch ein oder mehrere O-Atome unterbrochenes $C_3$-$C_{12}$-Alkyl, $C_5$-$C_8$-Cycloalkyl, Benzyl, Phenyl oder durch Halogen, $C_1$-$C_4$-Alkyl, $C_1$-$C_4$-Alkoxy oder Nitro substituiertes Phenyl bedeutet oder $-N(R^6)_2$ eine Pyrrolidino-, Piperidino- oder Morpholinogruppe bedeutet,
$R^7$ Wasserstoff oder $C_1$-$C_{20}$-Alkyl bedeutet, das durch Halogen oder Hydroxyl substituiert sein kann, oder $C_3$-$C_{20}$-Alkyl bedeutet, das durch ein oder mehrere Sauerstoffatome unterbrochen ist und durch Hydroxyl substituiert sein kann, und m 0, 1 oder 2 bedeutet.

**2.** Zusammensetzung gemäss Anspruch 1, dadurch gekennzeichnet, dass in Formel I und II ein R Wasserstoff, $C_1$-$C_4$-Alkyl, $C_1$-$C_4$-Alkoxy, Halogen, Trifluormethyl oder Nitro bedeutet und die anderen drei R Wasserstoff sind.

**3.** Zusammensetzung gemäss Anspruch 1, dadurch gekennzeichnet, dass in Formel I $R^1$ Wasserstoff, $C_1$-$C_8$-Alkyl, Phenyl oder Furyl bedeutet und $R^2$ Wasserstoff ist.

**4.** Zusammensetzung gemäss Anspruch 1, dadurch gekennzeichnet, dass in Formel I $R^1$ und $R^2$ Wasserstoff sind.

**5.** Zusammensetzung gemäss Anspruch 1, dadurch gekennzeichnet, dass in Formel I $R^3$ und $R^4$ unabhängig voneinander Wasserstoff, $C_1$-$C_8$-Alkyl, Allyl, $C_7$-$C_{10}$-Phenylalkyl, $C_1$-$C_4$-Alkoxy, Halogen, Phenyl, Cyclohexyl oder eine Gruppe $-CH_2CH_2COOR^7$ bedeutet, $R^5$ Wasserstoff, $C_1$-$C_{18}$-Alkyl, $C_3$-$C_{18}$-Alkenyl oder OH bedeutet und $R^7$ $C_1$-$C_{18}$-Alkyl ist oder $C_3$-$C_{20}$-Alkyl, das durch ein oder mehrere Sauerstoffatome unterbrochen ist.

**6.** Zusammensetzung gemäss Anspruch 1, enthaltend eine Verbindung der Formel III,

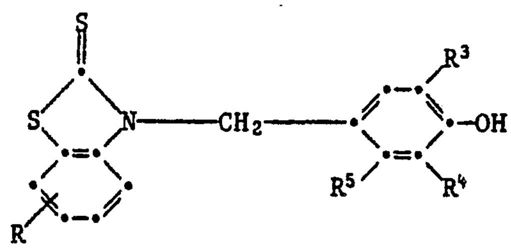

III

worin R Wasserstoff, $C_1$-$C_4$-Alkyl, $C_1$-$C_4$-Alkoxy, Chlor, Trifluormethyl oder Nitro ist, $R^3$ und $R^4$ unabhängig voneinander Wasserstoff, $C_1$-$C_8$-Alkyl, Allyl, Chlor, Methoxy, $C_7$-$C_{10}$-Phenylalkyl, Phenyl oder Cyclohexyl sind und $R^5$ Wasserstoff, Methyl oder OH ist.

7. Zusammensetzung gemäss Anspruch 1, enthaltend eine Verbindung der Formel IV,

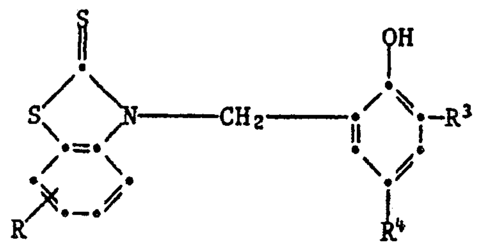

IV

worin R Wasserstoff, $C_1$-$C_4$-Alkyl, $C_1$-$C_4$-Alkoxy, Chlor, Trifluormethyl oder Nitro ist, $R^3$ und $R^4$ unabhängig voneinander Wasserstoff, $C_1$-$C_8$-Alkyl, $C_7$-$C_{10}$-Phenylalkyl, Phenyl, Cyclohexyl oder eine Gruppe der Formel IIa sind

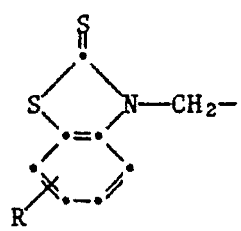

IIa

oder $R^3$ und $R^4$ zusammen einen anellierten Benzolring, Pyridinring oder Benzofuranring bilden.

8. Zusammensetzung gemäss Anspruch 1, welche ein Primer für metallische Substrate ist.

9. Zusammensetzung gemäss Anspruch 8, welche ein Primer auf Eisen, Stahl, Kupfer, Zink oder Aluminium ist.

10. Zusammensetzung gemäss Anspruch 1, welche ein wässriger Anstrichstoff ist.

11. Zusammensetzung gemäss Anspruch 10, welche ein kathodisch abscheidbarer Anstrichstoff ist.

12. Zusammensetzung gemäss Anspruch 1, enthaltend 0,5 bis 5 Gew.-%, bezogen auf den Feststoffgehalt des Anstrichstoffes, mindestens einer Verbindung der Formel I wie in Anspruch 1 definiert.

13. Anstrichstoff gemäss Anspruch 1 auf der Basis eines Epoxyharzes, eines Polyurethanes, eines Aminoplastes oder eines Acryl-, Alkydoder Polyesterharzes oder einer Mischung solcher Harze.

14. Anstrichstoff gemäss Anspruch 1 auf der Basis eines Vinylpolymeren, eines Celluloseesters, eines Chlorkautschuks, eines Phenolharzes, eines Styrol-Butadien-Copolymeren oder eines trocknenden Oeles.

15. Zusammensetzung gemäss Anspruch 1, welche ein Schmiermittel ist.

16. Schmiermittelzusammensetzung gemäss Anspruch 1, enthaltend 0,2 bis 2 Gew.-% einer Verbindung der Formel I wie in Anspruch 1 definiert.

17. Schmiermittelzusammensetzung gemäss Anspruch 15, enthaltend eine Verbindung der Formel IV wie in Anspruch 7 definiert.

18. Verfahren zur Herstellung von Verbindungen der Formel I in Anspruch 1, dadurch gekennzeichnet, dass man eine Verbindung der Formel V

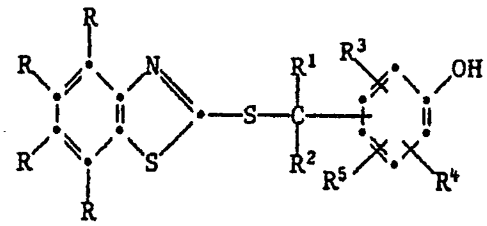

V

auf eine Temperatur von 70-250°, vorzugsweise 100-200° erhitzt.

19. Verfahren gemäss Anspruch 18, dadurch gekennzeichnet, dass man das Erhitzen in einem polaren Lösungsmittel ausführt.

20. Verfahren gemäss Anspruch 18, dadurch gekennzeichnet, dass man das Erhitzen in Gegenwart eines basischen Katalysators ausführt.

21. Verfahren zur Herstellung von Verbindungen der Formel I in Anspruch 1, dadurch gekennzeichnet, dass man ein 2-Mercaptobenzthiazol der Formel VI

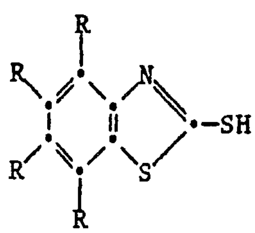

VI

und eine Carbonylverbindung

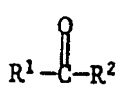

mit einem Phenol der Formel VIII

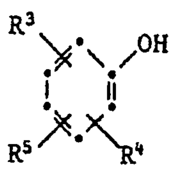

VIII

in Gegenwart eines basischen Katalysators umsetzt.

22. Verfahren gemäss Anspruch 21, dadurch gekennzeichnet, dass man die Reaktion in einem polaren

Lösungsmittel ausführt.

## Claims

1. A composition composed of a coating material or a lubricant, containing at least one compound of the formula I

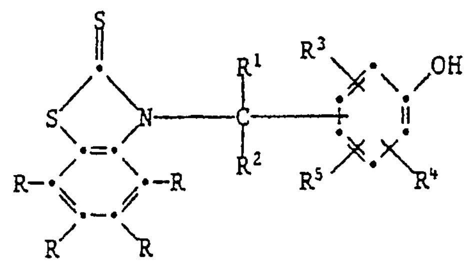

I

in which each R independently of one another is hydrogen, $C_1$-$C_{12}$alkyl, $C_1$-$C_4$haloalkyl, $C_1$-$C_{12}$alkoxy, $C_1$-$C_{12}$alkylthio, phenylthio, benzylthio, $C_1$-$C_{12}$alkylsulfonyl, phenyl, $C_7$-$C_{15}$alkylphenyl, $C_7$-$C_{10}$phenylalkyl, $C_5$-$C_8$cycloalkyl, halogen, $-NO_2$, -CN, -COOH, -COO-($C_1$-$C_4$alkyl), -OH, $-NH_2$ $-NHR^6$, $-N(R^6)_2$, $-CONH_2$, $-CONHR^6$ or $-CON(R^6)_2$,
$R^1$ is hydrogen, $C_1$-$C_{12}$alkyl, phenyl, phenyl which is substituted by halogen, $C_1$-$C_4$alkyl, $C_1$-$C_4$alkoxy or $-NO_2$, pyridyl, thienyl, or furyl,
$R^2$ is hydrogen or $C_1$-$C_4$alkyl,
$R^3$ and $R^4$ independently of one another are hydrogen, halogen, $C_1$-$C_4$alkoxy, cyano, nitro, $C_1$-$C_{20}$alkyl, $-(CH_2)_m-COOR^7$, $-(CH_2)_m-CONHR^6$, $-(CH_2)_m-CON(R^6)_2$, $C_3$-$C_{20}$alkenyl, $C_7$-$C_{10}$phenylalkyl, phenyl, cyclohexyl, cyclopentyl or a group of the formula II

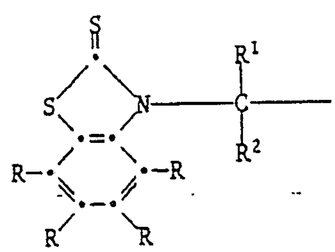

II

$R^5$ is hydrogen, $C_1$-$C_{20}$alkyl, $C_3$-$C_{20}$alkenyl or hydroxy, or $R^3$ and $R^5$together or $R^4$ and $R^5$ together form a ring fused to the phenol ring and which may be a carbocyclic or heterocyclic ring which may contain oxygen, nitrogen or sulfur as heteroatoms and which may be substituted by $C_1$-$C_4$alkyl, $C_1$-$C_4$alkoxy or halogen, $R^6$ is $C_1$-$C_{12}$alkyl, $C_3$-$C_{12}$alkyl which is interrupted by one or more O atoms, $C_5$-$C_8$cycloalkyl, benzyl, phenyl, or phenyl which is substituted by halogen, $C_1$-$C_4$alkyl, $C_1$-$C_4$alkoxy or nitro, or $-N(R^6)_2$ is a pyrrolidino, piperidino or morpholino group, $R^7$ is hydrogen or $C_1$-$C_{20}$alkyl, which may be substituted by halogen or hydroxyl or $R^7$ is $C_3$-$C_{20}$alkyl which is interrupted by one or more oxygen atoms and may be substituted by hydroxyl and m is 0, 1 or 2.

2. A composition according to claim 1, wherein, in formula I and II, one R is hydrogen, $C_1$-$C_4$alkyl, $C_1$-$C_4$alkoxy, halogen, trifluoromethyl or nitro and the other three R's are hydrogen.

3. A composition according to claim 1, wherein, in formula I, $R^1$ is hydrogen, $C_1$-$C_8$alkyl, phenyl or furyl and $R^2$ is hydrogen.

4. A composition according to claim 1, wherein $R^1$ and $R^2$ in formula I are hydrogen.

5. A composition according to claim 1, wherein, in formula I, $R^3$ and $R^4$ independently of one another are hydrogen, $C_1$-$C_8$alkyl, allyl, $C_7$-$C_{10}$-phenylalkyl, $C_1$-$C_4$alkoxy, halogen, phenyl, cyclohexyl, or a group $-CH_2CH_2COOR^7$, $R^5$ is hydrogen, $C_1$-$C_{18}$alkyl, $C_3$-$C_{18}$alkenyl or OH and $R^7$ is $C_1$-$C_{18}$alkyl or $C_3$-

$C_{20}$alkyl which is interrupted by one or more oxygen atoms.

6. A composition according to claim 1, containing a compound of the formula III

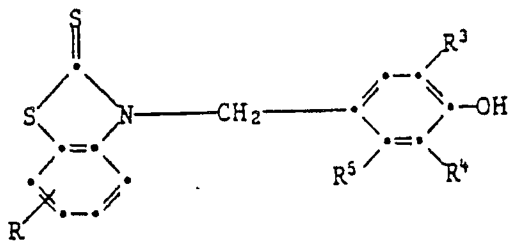

III

in which R is hydrogen, $C_1$-$C_4$alkyl, $C_1$-$C_4$alkoxy, chlorine, trifluoromethyl or nitro, $R^3$ and $R^4$ independently of one another are hydrogen, $C_1$-$C_8$alkyl, allyl, chlorine, methoxy, $C_7$-$C_{10}$phenylalkyl, phenyl or cyclohexyl and $R^5$ is hydrogen, methyl or OH.

7. A composition according to claim 1, containing a compound of the formula IV

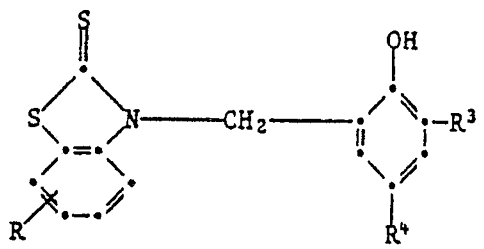

IV

in which R is hydrogen, $C_1$-$C_4$alkyl, $C_1$-$C_4$alkoxy, chlorine, trifluoromethyl or nitro, $R^3$ and $R^4$ independently of one another are hydrogen, $C_1$-$C_8$alkyl, $C_7$-$C_{10}$phenylalkyl, phenyl, cyclohexyl or a group of the formula IIa

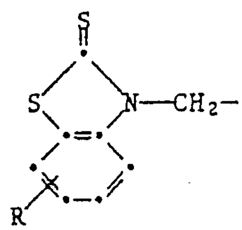

IIa

or $R^3$ and $R^4$ together form a fused benzene ring, pyridine ring or benzofuran ring.

8. A composition according to claim 1 which is a primer for metallic substrates.

9. A composition according to claim 8 which is a primer on iron, steel, copper, zinc or aluminium.

10. A composition according to claim 1 which is an aqueous coating material.

11. A composition according to claim 10 which is a coating material which can be cathodically deposited.

12. A composition according to claim 1 containing 0.5 to 5% by weight, based on the solids content of the coating material, of at least one compound of the formula I as defined in claim 1.

13. A coating material according to claim 1 on the basis of an epoxy resin, a polyurethane, an aminoplastic, or an acrylic, alkyd or polyester resin or a mixture of such resins.

14. A coating material according to claim 1 on the basis of a vinyl polymer, a cellulose ester, a chlorinated

rubber, a phenolic resin, a styrene-butadiene copolymer or a drying oil.

**15.** A composition according to claim 1 which is a lubricant.

**16.** A lubricant composition according to claim 1 containing 0.2 to 2% by weight of a compound of the formula I as defined in claim 1.

**17.** A lubricant composition, according to claim 15 containing a compound of the formula IV as defined in claim 7.

**18.** A compound of the formula I

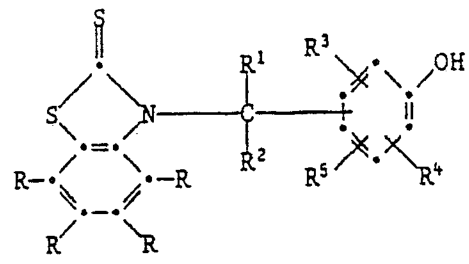

I

in which each R independently of one another is hydrogen, $C_1$-$C_{12}$alkyl, $C_1$-$C_4$haloalkyl, $C_1$-$C_{12}$alkoxy, $C_1$-$C_{12}$alkylthio, phenylthio, benzylthio, $C_1$-$C_{12}$alkylsulfonyl, phenyl, $C_7$-$C_{15}$alkylphenyl, $C_7$-$C_{10}$phenylalkyl, $C_5$-$C_8$cycloalkyl, halogen, $-NO_2$, -CN, -COOH, -COO-($C_1$-$C_4$a1ky1), -OH, $-NH_2$ $-NHR^6$, $-N(R^6)_2$, $-CONH_2$, $-CONHR^6$ or $-CON(R^6)_2$,
$R^1$ is hydrogen, $C_1$-$C_{12}$alkyl, phenyl, phenyl which is substituted by halogen, $C_1$-$C_4$alkyl, $C_1$-$C_4$alkoxy or $-NO_2$, pyridyl, thienyl, or furyl,
$R^2$ is hydrogen or $C_1$-$C_4$alkyl,
$R^3$ and $R^4$ independently of one another are hydrogen, halogen, $C_1$-$C_4$alkoxy, cyano, nitro, $C_1$-$C_{20}$alkyl, $-(CH_2)_m-COOR^7$, $-(CH_2)_m-CONHR^6$, $-(CH_2)_m-CON(R^6)_2$, $C_3$-$C_{20}$alkenyl, $C_7$-$C_{10}$phenylalkyl, phenyl, cyclohexyl, cyclopentyl or a group of the formula II

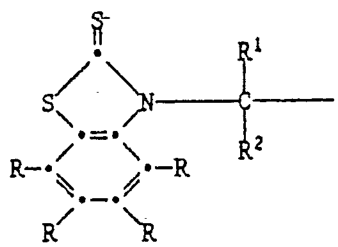

II

$R^5$ is hydrogen, $C_1$-$C_{20}$alkyl, $C_3$-$C_{20}$alkenyl or hydroxy, or $R^3$ and $R^5$ together or $R^4$ and $R^5$ together form a ring fused to the phenol ring and which may be a carbocyclic or heterocyclic ring which may contain oxygen, nitrogen or sulfur as heteroatoms and which may be substituted by $C_1$-$C_4$alkyl, $C_1$-$C_4$alkoxy or halogen, R6 is $C_1$-$C_{12}$alkyl, $C_3$-$C_{12}$alkyl which is interrupted by one or more O atoms, $C_5$-$C_8$cycloalkyl, benzyl, phenyl, or phenyl which is substituted by halogen, $C_1$-$C_4$alkyl, $C_1$-$C_4$alkoxy or nitro, or $-N(R^6)_2$ is a pyrrolidino, piperidino or morpholino group, $R^7$ is hydrogen or $C_1$-$C_{20}$alkyl, which may be substituted by halogen or hydroxyl or $R^7$ is $C_3$-$C_{20}$alkyl which is interrupted by one or more oxygen atoms and may be substituted by hydroxyl and m is O, 1 or 2, subject to the proviso that, if R, $R^1$ and $R^2$ are hydrogen and $R^3$ and $R^4$ are tert-butyl groups in the ortho-position relative to the hydroxyl group, $R^5$ is not hydrogen.

**19.** A process for the preparation of compounds of the formula I in claim 1, wherein a compound of the formula V

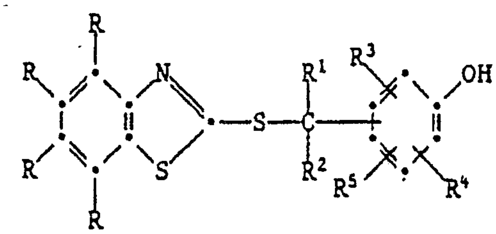

V

is heated at a temperature of 70 - 250°, preferably 100 - 200°.

**20.** A process according to claim 19, wherein heating is carried out in a polar solvent.

**21.** A process according to claim 19, wherein heating is carried out in the presence of a basic catalyst.

**22.** A process for the preparation of compounds of the formula I in claim 1, wherein a 2-mercaptobenzothiazole of the formula VI

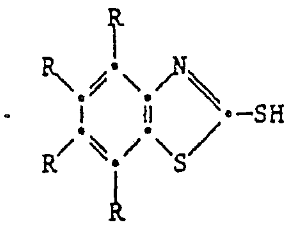

VI

and a carbonyl compound

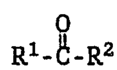

are reacted with a phenol of the formula VIII

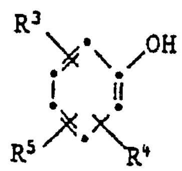

VIII

in the presence of a basic catalyst.

**23.** A process according to claim 22, wherein the reaction is carried out in a polar solvent.

Claims for the following Contracting States : AT, ES

**1.** A composition composed of a coating material or a lubricant, containing at least one compound of the formula I

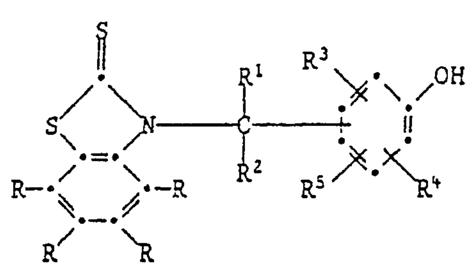

I

in which each R independently of one another is hydrogen, $C_1$-$C_{12}$alkyl, $C_1$-$C_4$haloalkyl, $C_1$-$C_{12}$alkoxy,

$C_1$-$C_{12}$alkylthio, phenylthio, benzylthio, $C_1$-$C_{12}$alkylsulfonyl, phenyl, $C_7$-$C_{15}$alkylphenyl, $C_7$-$C_{10}$phenylalkyl, $C_5$-$C_8$cycloalkyl, halogen, $-NO_2$ -CN, -COOH, -COO-($C_1$-$C_4$alkyl), -OH, $-NH_2$ $-NHR^6$, $-N(R^6)_2$, $-CONH_2$, $-CONHRA^6$ or $-CON(R^6)_2$,

$R^1$ is hydrogen, $C_1C_{12}$alkyl, phenyl, phenyl which is substituted by halogen, $C_1$-$C_4$alkyl, $C_1$-$C_4$alkoxy or $-NO_2$, pyridyl, thienyl, or furyl,

$R^2$ is hydrogen or $C_1$-$C_4$alkyl,

$R^3$ and $R^4$ independently of one another are hydrogen, halogen, $C_1$-$C_4$alkoxy, cyano, nitro, $C_1$-$C_{20}$alkyl, $(CH_2)_m$-$COOR^7$, $-(CH_2)_m$-$CONHR^6$, $-(CH_2)_m$-$CON(R^6)_2$, $C_3$-$C_{20}$alkenyl, $C_7$-$C_{10}$phenylalkyl, phenyl, cyclohexyl, cyclopentyl or a group of the formula II

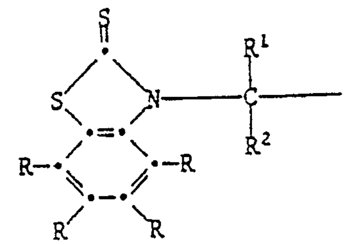

II

$R^5$ is hydrogen, $C_1$-$C_{20}$alkyl, $C_3$-$C_{20}$alkenyl or hydroxy, or $R^3$ and $R^5$ together or $R^4$ and $R^5$ together form a ring fused to the phenol ring and which may be a carbocyclic or heterocyclic ring which may contain oxygen, nitrogen or sulfur as heteroatoms and which may be substituted by $C_1$- $C_4$alkyl, $C_1$-$C_4$alkoxy or halogen, $R^6$ is $C_1$-$C_{12}$alkyl, $C_3$-$C_{12}$alkyl which is interrupted by one or more O atoms, $C_5$-$C_8$cycloalkyl, benzyl, phenyl, or phenyl which is substituted by halogen, $C_1$-$C_4$alkyl, $C_1$-$C_4$alkoxy or nitro, or $-N(R^6)_2$ is a pyrrolidino, piperidino or morpholino group, $R^7$ is hydrogen or $C_1$-$C_{20}$alkyl, which may be substituted by halogen or hydroxyl or $R^7$ is $C_3$ - $C_{20}$alkyl which is interrupted by one or more oxygen atoms and may be substituted by hydroxyl and m is 0, 1 or 2.

**2.** A composition according to claim 1, wherein, in formula I and II, one R is hydrogen, $C_1$-$C_4$alkyl, $C_1$-$C_4$alkoxy, halogen, trifluoromethyl or nitro and the other three R's are hydrogen.

**3.** A composition according to claim 1, wherein, in formula I, $R^1$ is hydrogen, $C_1$-$C_8$alkyl, phenyl or furyl and $R^2$ is hydrogen.

**4.** A composition according to claim 1, wherein $R^1$ and $R^2$ in formula I are hydrogen.

**5.** A composition according to claim 1, wherein, in formula I, $R^3$ and $R^4$ independently of one another are hydrogen, $C_1$-$C_8$alkyl, allyl, $C_7$-$C_{10}$-phenylalkyl, $C_1$-$C_4$alkoxy, halogen, phenyl, cyclohexyl, or a group $-CH_2CH_2COOR^7$, $R^5$ is hydrogen, $C_1$-$C_{18}$alkyl, $C_3$-$C_{18}$alkenyl or OH and $R^7$ is $C_1$-$C_{18}$alkyl or $C_3$-$C_{20}$alkyl which is interrupted by one or more oxygen atoms.

**6.** A composition according to claim 1, containing a compound of the formula III

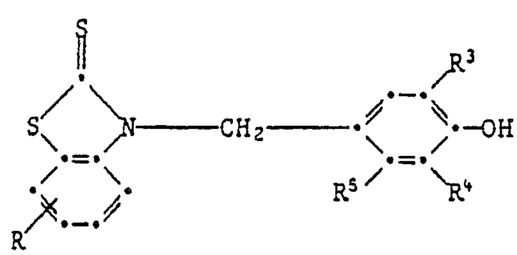

III

in which R is hydrogen, $C_1$-$C_4$alkyl, $C_1$-$C_4$alkoxy, chlorine, trifluoromethyl or nitro, $R^3$ and $R^4$ independently of one another are hydrogen, $C_1$-$C_8$alkyl, allyl, chlorine, methoxy, $C_7$-$C_{10}$phenylalkyl, phenyl or cyclohexyl and $R^5$ is hydrogen, methyl or OH.

**7.** A composition according to claim 1, containing a compound of the formula IV

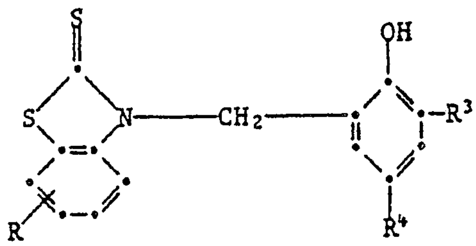

IV

in which R is hydrogen, $C_1$-$C_4$alkyl, $C_1$-$C_4$alkoxy, chlorine, trifluoromethyl or nitro, $R^3$ and $R^4$ independently of one another are hydrogen, $C_1$-$C_8$alkyl, $C_7$-$C_{10}$phenylalkyl, phenyl, cyclohexyl or a group of the formula IIa

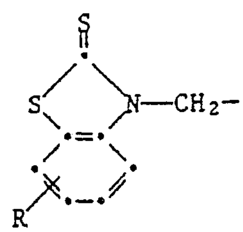

IIa

or $R^3$ and $R^4$ together form a fused benzene ring, pyridine ring or benzofuran ring.

**8.** A composition according to claim 1 which is a primer for metallic substrates.

**9.** A composition according to claim 8 which is a primer on iron, steel, copper, zinc or aluminium.

**10.** A composition according to claim 1 which is an aqueous coating material.

**11.** A composition according to claim 10 which is a coating material which can be cathodically deposited.

**12.** A composition according to claim 1 containing 0.5 to 5% by weight, based on the solids content of the coating material, of at least one compound of the formula I as defined in claim 1.

**13.** A coating material according to claim 1 on the basis of an epoxy resin, a polyurethane, an aminoplastic, or an acrylic, alkyd or polyester resin or a mixture of such resins.

**14.** A coating material according to claim 1 on the basis of a vinyl polymer, a cellulose ester, a chlorinated rubber, a phenolic resin, a styrene-butadiene copolymer or a drying oil.

**15.** A composition according to claim 1 which is a lubricant.

**16.** A lubricant composition according to claim 1 containing 0.2 to 2% by weight of a compound of the formula I as defined in claim 1.

**17.** A lubricant composition, according to claim 15 containing a compound of the formula IV as defined in claim 7.

**18.** A process for the preparation of compounds of the formula I in claim 1, wherein a compound of the formula V

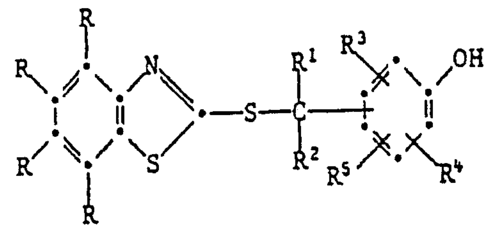

V

is heated at a temperature of 70 - 250°, preferably 100 - 200°.

**19.** A process according to claim 18, wherein heating is carried out in a polar solvent.

**20.** A process according to claim 18, wherein heating is carried out in the presence of a basic catalyst.

**21.** A process for the preparation of compounds of the formula I in claim 1, wherein a 2-mercaptobenzothiazole of the formula VI

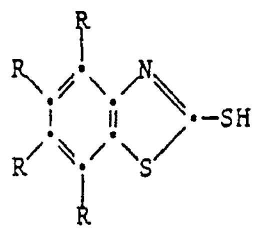

VI

and a carbonyl compound

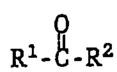

are reacted with a phenol of the formula VIII

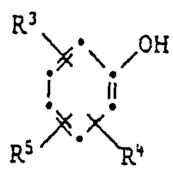

VIII

in the presence of a basic catalyst.

**22.** A process according to claim 21, wherein the reaction is carried out in a polar solvent.

**Revendications**

**1.** Composition constituée d'un produit de revêtement ou d'un lubrifiant, composition qui contient au moins un composé répondant à la formule I :

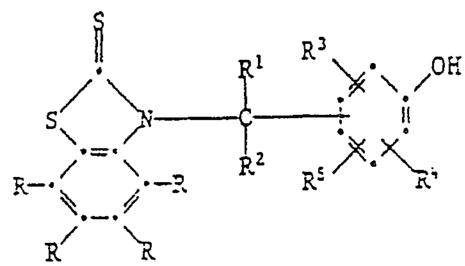

(I)

dans laquelle

les R

représentent chacun, indépendamment les uns des autres, l'hydrogène, un alkyle en $C_1$-$C_{12}$, un halogénoalkyle en $C_1$-$C_4$, un alcoxy en $C_1$-$C_{12}$ un alkylthio en $C_1$-$C_{12}$, un phénylthio, un benylthio, un alkylsulfonyle en $C_1$-$C_{12}$, un phényle, un alkylphényle en $C_7C_{15}$, un phénylalkyle en $C_7$-$C_{10}$, un cycloalxyle en $C_5$-$C_8$, un halogène, $NO_2$, -CN, -COOH, -COO($C_1$-$C_4$-alkyl), -OH, $-NH_2$, $-NHR^6$, $-N(R^6)_2$, $-CONH_2$, $-CONHR^6$ ou $-CON(R^6)_2$,

$R^1$

représente l'hydrogène, un alkyle en $C_1$-$C_2$, un phényle, un phényle porteur d'un halogène, d'un alkyle en $C_1$-$C_4$, d'un alcoxy en $C_1$-$C_4$ ou d'un $-NO_2$, un pyridyle, un thiényle ou un furyle,

$R^2$

représente l'hydrogène ou un alkyle en $C_1$-$C_4$,

$R^3$ et $R^4$

représentent chacun, indépendamment l'un de l'autre, l'hydrogène, un halogéne, un alcoxy en $C_1$-$C_4$, un cyano, un nitro, un alkyle en $C_1$-$C_{20}$, $-(CH_2)_m-COOR^7$, $(CH_2)CONHR^6$, $-(CH_2)_mCON(R^6)_2$, un alcényle en $C_3$-$C_{20}$, un phénylalkyle en $C_7$-$C_{10}$, un phényle, un cyclohexyle, un cyclopentyle ou un radical de formule II :

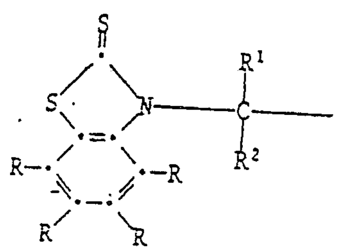

(II),

$R^5$

représente l'hydrogène, un alkyle en $C_1$-$C_{20}$, un alcényle en $C_3$-$c_{20}$ ou un hydroxy, ou

$R^3$ et $R^5$

ensemble, ou $R^4$ et $R^5$ ensemble, forment un cycle qui est condensé au cycle du phénol et qui peut être un cycle carbocyclique ou hétérocyclique, ce cycle pouvant contenir, comme hétéroatomes, des atomes d'oxygène, d'azote ou de soufre et pouvant porter, comme substituant (S), un alkyle en $C_1$-$C_4$, un alcoxy en $C_1$-$C_4$ ou un halogène,

$R^6$

représente un alkyle en $C_1$-$C_{12}$, un alkyle en $C_3$-$C_{12}$ interrompu par un ou plusieurs atomes d 'oxygène, un cycloalkyle en $C_5$-$C_8$, un benzyle, un phényle ou un pnényle porteur d'un halogène, d'un alkyle en $C_1$-$C_4$, d'un alcoxy en $C_1$-$C_4$ ou d'un nitro, ou $-N(R^6)_2$ représente un radical pyrrolidino, pipéridino ou morpholino,

$R^7$

représente l'hydrogène, un alkyle en $C_1$-$C_{20}$ éventuellement porteur d'un halogène ou d'un hydroxy, ou un alkyle en $C_3$-$C_{20}$ qui est interrompu par un ou plusieurs atomes d'oxygène et qui peut porter un hydroxy, et

m

est égal à 0, à 1 ou à 2.

2. Composition selon la revendication 1 caractérisée en ce que, dans les formules I et II, l'un des

symooles R représente l'hydrogène, un alkyle en $C_1$-$C_4$, un alcoxy en $c_1$-$c_4$, un halogène, un trifluorométhyle ou un nitro, et les trois autres R représentent chacun l'hydrogène.

3. Composition selon la revendication 1 caractérisée en ce que, dans la formule I, $R^1$ représente l'hydrogène, un alkyle en $C_1$-$C_8$, un phényle ou un furyle et $R^2$ représente l'hydrogène.

4. Composition selon la revendication 1 caractérisée en ce que, dans la formule I, $R^1$ et $R^2$ représentent chacun l'hydrogène.

5. Composition selon la revendication 1 caractérisée en ce que, dans la formule I $R^3$ et $R^4$ représentent chacun, indépendamment l'un de l'autre, l'hydrogène, un alkyle en $C_1$-$C_8$, un allyle, un phénylalkyle en $C_7$-$C_{10}$, un alcoxy en $C_1$-$C_4$, un halogène, un phényle₁ un cyclohexyle ou un radical $-CH_2CH_2COOR^7$, $R^5$ représente l'hydrogène, un alkyle en $C_1$-$C_{18}$, un alcényle en $C_3$-$C_{18}$ ou OH, et $R^7$ représente un alkyle en $C_1$-$C_{19}$ ou un alkyle en $C_3$-$C_{20}$ qui est interrompu par un ou plusieurs atomes d'oxygène.

6. Composition selon la revendicaticn 1 qui contient un composé répondant à la formule III :

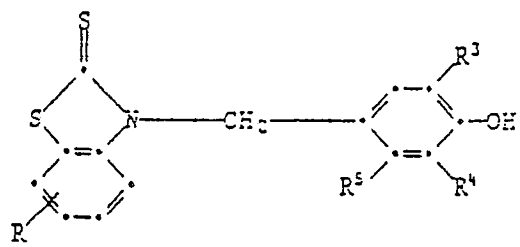

(III)

dans laquelle
R
représente l'hydrogene, un alkyle en $C_1$-$C_4$, un alcoxy en $C_1$-$C_4$, un chlore, un trifluorométhyle ou un nitro,
$R^3$ et $R^4$
représentent chacun, indépendamment l'un de l'autre, l'hydrogène, un alkyle en $C_1$-$C_8$, un allyle, un chlore, un méthoxy, un phénylalkyle en $C_7$-$C_{10}$, un phényle ou un cyclohexyle et
$R^5$
représente l'hydrogène, un méthyle ou OH.

7. Composition selon la revendicaticn 1 qui contient un composé répondant à la formule IV :

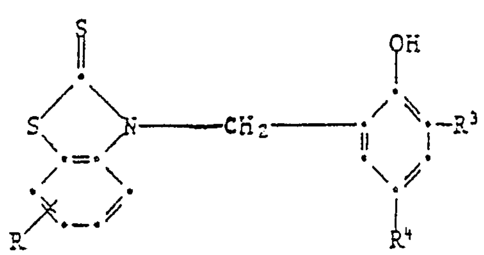

(IV)

dans laquelle
R
représente l'hydrogène, un alkyle en $C_1$-$C_4$, un alcoxy en $C_1$-$C_4$, un chlore, un trifluorométhyle ou un nitro,
$R^3$ et $R^4$
représentent chacun, indépendamment l'un de l'autre, l'hydrogène, un alkyle en $C_1$-$C_8$, un phénylalkyle en $C_7$-$C_{10}$, un phényle, un cyclohexyle ou un radical de formule IIa :

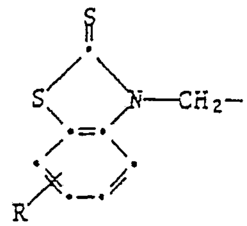

(IIa)

ou $R^3$ et $R^4$
forment ensemole un cycle condensé de benzène, de pyridine ou de benzofuranne.

**8.** Composition selon la revendica'ion 1 qui es une peinture de fond pour substrates métalliques.

**9.** Composition selon la revendication 8 qui est une peinture de fond pour fer, acier, cuivre, zinc ou aluminium.

**10.** Composition selon la revendication 1 qui est un produit de revêtement aqueux.

**11.** Composition selon la revendication 10 qui est un produit de revêtement applicable cathodiquement.

**12.** Composition selon la revendication 1 qui contient de 0,5 à 5 % en poids, par rapport à la matière solide du produit de revêtement, d'au moins un composé de formule I selon la revendicatlon 1.

**13.** Produit de revêtement selon la revendication 1 à base d'une résine époxydique, d'un polyuréthanne, d'un aminoplaste, d'une résine acrylique, d'alkydes ou de polyesters, ou d'un mélange de telles résines.

**14.** Produit de revêtement selon la revendication 1 à base d'un polymère vinylique, d'un ester de la cellulose, d'un caoutchouc chloré, d'une résine phénolique, d'un copolymère styrène-butadiène ou d'une huile siccative.

**15.** Composition selon la revendication 1 qui est un lubrifiant.

**16.** Composition lubrifiante selon la revendication 1 qui contient de 0,2 à 2 % en poids d'un composé de formule I tel que défini à la revendication 1.

**17.** Composition lubrifiante selon la revendication 15 qui contient un composé de formule IV tel que défini à la revendication 7.

**18.** Composé répondant à la formule I :

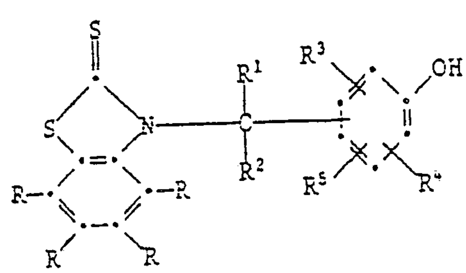

(I)

dans laquelle
les R
représentent chacun, indépendamment les uns des autres, l'hydrogène, un alkyle en $C_1$-$C_{12}$, un halogénoalkyle en $C_1$-$C_4$, un alcoxy en $C_1$-$C_{12}$, un alkylthio en $C_1$-$C_{12}$, un phénylthio, un benzylthio, un alkylsulfonyle en $C_1$-$C_{12}$, un phényle, un alylphényle en $C_7$–$C_{15}$, un phénylalkyle en $C_7$-$C_{10}$, un

cycloalkyle en $C_5$-$C_8$, un haloaène $-NO_2$, -CN, -COOH, -COO ($C_1$-$C_4$-alkyl) -OH, $-NH_2$, $-NHR^6$, -N $(R^6)_2$, $-CONH_2$, $-CONHR^6$ ou $-CON(R^6)_2$,

$R^1$

représente l'hydrogène, un alkyle en $C_1$-$C_{12}$, un phényle, un phényle porteur d'un halogène, d'un alkyle en $C_1$-$C_4$, d'un alcoxy en $C_1$-$C_4$ ou d'un $-NO_2$, un pyridyle, un thiényle ou un furyle,

$R^2$

représente l'hydrogène ou un alkyle en $C_1$-$C_4$,

$R^3$ et $R^4$

représentent chacun, indépendamment l'un de l'autre, l'hydrogène, un halogène, un alcoxy en $C_1$-$C_4$, un cyano, un nitro, un alkyle en $C_1$-$C_{20}$, - $(CH_2)_m$ - $COOR^7$, $(Ch_2)_m$-$CONHR^6$, $(CH_2)_m$-$CON(R^6)_2$, un alcényle en $C_3$-$C_{20}$, un phénylalkyle en $C_7$-$C_{10}$, un phénle, un cycloheyle, un cyclopentyle ou un radical de formule II

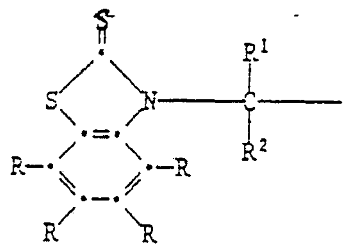

(II),

$R^5$

représente l'hydrogène, un alkyle en $C_1$-$C_{20}$, un alcényle en $C_3$-$C_{20}$ ou un hydroxy, ou

$R^3$ et $R^5$

ensemble, ou $R^4$ et $R^5$ ensemble, forment un cycle qui est condensé au cycle du phénol et qui peut être un cycle carbocyclique ou hétérocyclique, ce cycle pouvant contenir, comme hétéroatomes, des atomes d'oxygène, d'azote ou de soufre et pouvant porter, comme substituant (s), un alkyle en $C_1$-$C_4$, un alcoxy en $C_1$-$C_4$ ou un halogène,

$R^6$

représente un alkyle en $C_1$-$C_{12}$, un alkyle en $C_3$-$C_{12}$ interrompu par un ou plusieurs atomes d'oxygène, un cycloalkyle en $C_5$-$C_8$, un benzyle, un phényle ou un phényle porteur d'un halogène, d'un alxyle en $C_1$-$C_4$, d'un alcoxy en $C_1$-$C_4$ ou d'un nitro, ou $-N(R^6)_2$ représente un radical pyrrolidino, pipéridino ou morpholino,

$R^7$

représente l'hydrogène, un alkyle en $C_1$-$C_{20}$ éventuellement porteur d'un halogène ou d'un hydroxy, ou un alkyle en $C_3$-$C_{30}$ qui est interrompu par un ou plusieurs atomes d'oxygène et qui peut porter un hydroxy, et

m

est égal à 0, à 1 ou à 2,

avec la condition que, lorsque R, $R^1$ et $R^2$ représentent chacun l'hydrogène et que $R^3$ et $R^4$ représentent des radicaux tertbutyles en position ortho par rapport au radical hydroxy$\gamma$, $R^5$ ne représente pas l'hydrogène.

**19.** Procédé pour préparer ces composés de formule I selon la revendication 1, procédé caractérisé en ce qu'on chauffe un composé de formule V :

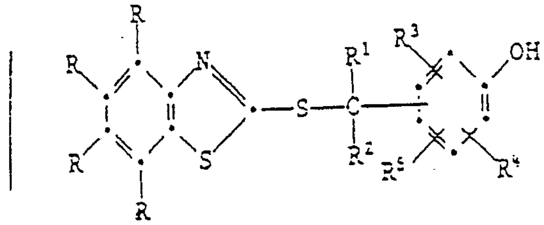

(V)

à une température de 70 à 250°, de préférence de 100 à 200°.

**20.** Procédé selon la revendication 19 caractérisé en ce qu'on effectue le chauffage dans un solvant polaire.

**21.** Procédé selon la revendication 19 caractérisé en ce qu'on effectue le chauffage en présence d'un catalyseur basique.

**22.** Procédé pour préparer des composés de formule I selon la revendication 1, procédé caractérisé en ce qu'on fait réagir le mercapto-2 benzothaizole de formule VI :

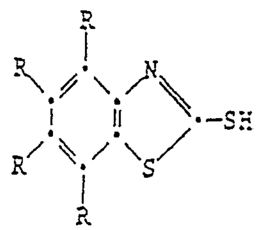

(VI)

et un composé carbonylique

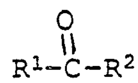

avec un phénol de formule VIII :

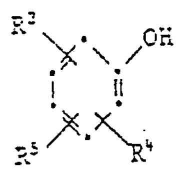

(VIII)

en présence d'un catalyseur basique.

**23.** Procédé selon la revendication 22 caractérisé en ce qu'on effectue la réaction dans un solvant polaire.

Revendications pour les Etats contractants suivants : AT, ES

**1.** Composition constituée d'un produit de revêtement ou d'un lubrifiant, composition qui contient au moins un composé répondant à la formule I :

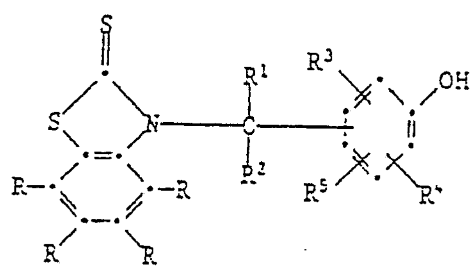

(I)

dans laquelle

les R

représentent chacun, indépendamment les uns des aures, l'hydrogène un alkyle en $C_1$-$C_{12}$, un halogénoalkyle en $C_1$-$C_4$, un alcoxy en $C_1$-$C_{12}$, un alkylthio en $C_1$-$C_{12}$, un phénylthio, un benzylthio, un alkylsulfonyle en $C_1$-$C_{12}$, un phényle, un alkylphényle en $C_7$-$C_{15}$, un phénylalkyle en $C_7$-$C_{10}$, un

cycloalkyle en $C_5$-$C_8$, un halogène, -$NO_2$ -CN, -COOH, -COO($C_1$-$C_4$-alkyl), -OH, $NH_2$, -$NHR^6$, -$N(R^6)_2$, -$CONH_2$, -$CONHR^6$ ou -$CON(R^6)_2$,

$R^1$

représente l'hydrogène, un alkyle en $C_1C_{12}$, un phényle, un phényle porteur d'un halogène, d'un alyle en $C_1$-$C_4$, d'un alcoxy en $C_1$-$C_4$ ou d'un -$NO_2$, un pyridyle, un thiényle ou un furyle,

$R^2$

représente l'hydrogène ou un alkyle en $C_1$-$C_4$,

$R^3$ et $R^4$

représentent chacun, indépendamment l'un de l'autre, l'hydrogène, un halogène, un alcoxy en $C_1$-$C_4$, un cyano un nitro, un alkyle en $C_1$-$C_{20}$, - $(CH_2)_m$ - $COOR,^7$ $(CH_2)_m$-$CONHR^6$,- $(CH_2)_m$-$CON(R^6)_2$, un alcényle en $C_3$-$C_{20}$ , un phénylalkyle en $C_7$-$C_{10}$, un phényle, un cyclohexyle, un cyclopentyle ou un radical de formule II :

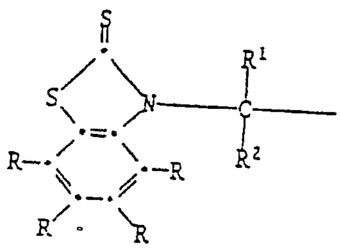

(II),

$R^5$

représente l'hydrogène, un alkyle en $C_1$-$C_{20}$, un alcényle en $C_3$-$C_{20}$ ou un hydroxy, ou

$R^3$ et $R^5$

ensemble, ou $R^4$ et $R^5$ ensemble, forment un cycle qui est condensé au cycle du phénol et qui peut être un cycle carbocyclique ou hétérocyclique, ce cycle pouvant contenir, comme hétéroatomes, des atomes d'oygène, d'azote ou de soufre et pouvant porter, comme substituant (s), un alkyle en $C_1$-$C_4$, un alcoxy en $C_1$-$C_4$ ou un halogène,

$R^6$

représente un alkyle en $C_1$-$C_{12}$, un alkyle en $C_3$-$C_{12}$ interrompu par un ou plusieurs atomes d'oxygène, un cycloalkyle en $C_5$-$C_8$, un benzyle, un phényle ou un phényle porteur d'un halogène, d'un alkyle en $C_1$-$C_4$, d'un alcoxy en $C_1$-$C_4$ ou d'un nitro, ou -$N(R^6)_2$ représente un radical pyrrolidino, pipéridino ou morpholino,

$R^7$

représente l'hydrogène, un alkyle en $C_1$-$C_{20}$ éventuellement porteur d'un halogène ou d'un hydroxy, ou un alkyle en $C_3$-$C_{20}$ qui est interrompu par un ou plusieurs atomes d'oxygène et qui peut porter un hydroxy, et

m

est égal à 0, à 1 ou à 2.

2. Composition selon la revendication 1 caractérisée en ce que, dans les formules I et II, l'un des symboles R représente l'hydrogène, un alkyle en $C_1$-$C_4$ un alcoxy en $C_1$-$C_4$, un halogène, un trifluorométhyle ou un nitro, et les trois autres R représentent chacun l'hydrogène.

3. Composition selon la revendication 1 caractérisée en ce que, dans la formule I, $R^1$ représente l'hydrogène, un alkyle en $C_1$-$C_8$, un phényle ou un furyle et $R^2$ représente l'hydrogène.

4. Composition selon la revendication 1 caractérisée en ce que, dans la formule I, $R^1$ et $R^2$ représentent chacun l'hydrogène.

5. Composition selon la revendication 1 caractérisée en ce que, dans la formule I $R^3$ et $R^4$ représentent chacun, indépendamment l'un de l'autre, l'hydrogène, un alkyle en $C_1$-$C_8$, un allyle, un phénylalkyle en $C_7$-$C_{10}$, un alcoxy en $C_1$-$C_4$, un halogène, un phényle, un cyclohexyle ou un radical -$CH_2CH_2COOR^7$, $R^5$ représente l'hydrogène, un alkyle en $C_1$-$C_{18}$, un alcényle en $C_3$-$C_{18}$ ou OH, et $R^7$ représente un alkyle en $C_1$-$C_{18}$ ou un alkyle en $C_3$-$C_{20}$ qui est interrompu par un ou plusieurs atomes d'oxygène.

6. Composition selon la revendication 1 qui contient un composé répondant à la formule III :

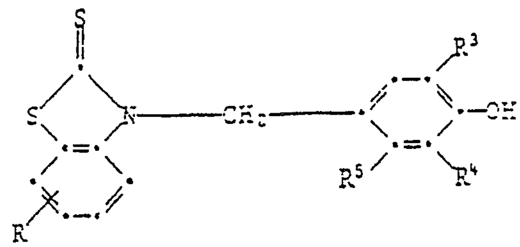

(III)

dans laquelle

R

représente l'hydrogène, un alkyle en $C_1$-$C_4$, un alcoxy en $C_1$-$C_4$, un chlore, un trifluorométhyle ou un nitro,

$R^3$ et $R^4$

représentent chacun, indépendamment l'un de l' autre, l'hydrogène, un alkyle en $C_1$-$C_8$ un allyle, un chlore, un méthoxy, un phénylalkyle en $C_7$-$C_{10}$, un phényle ou un cyclohexyle et

$R^5$

représente l'hydrogène, un méthyle ou OH.

7. Composition selon la revendication 1 qui contient un composé répondant à la formule IV :

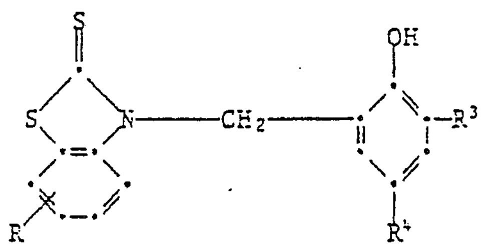

(IV)

dans laquelle

R

représente l'hydrogène, un alkyle en $C_1$-$C_4$, un alcoxy en $C_1$-$C_4$, un chlore, un trifluorométhyle ou un nitro,

$R^3$ et $R^4$

représentent chacun, indépendamment l'un de l'autre, l'hydrogène, un alkyle en $C_1$-$C_8$ un phénylalkyle en $C_7$-$C_{10}$, un phényle, un cyclohexyle ou un radical de formule IIa :

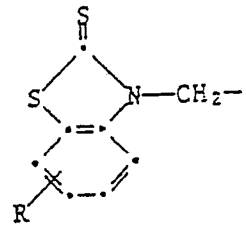

(IIa)

ou $R^3$ et $R^4$

forment ensemble un cycle condensé de benzène, de pyridine ou de benzofuranne.

8. Composition selon la revendication 1 qui est une peinture de fond pour substrats métalliques.

9. Composition selon la revendication 8 qui est une peinture de fond pour fer, acier, cuivre, zinc ou aluminium.

**10.** Composition selon la revendication 1 qui est un produit de revêtement aqueux.

**11.** Composition selon la revendication 10 qui est un produit de revêtement applicable cathodiquement.

**12.** Composition selon la revendication 1 qui contient de 0,5 à 5 % en poids, par rapport à la matière solide du produit de revêtement, d'au moins un composé de formule I selon la revendication 1.

**13.** Produit de revêtement selon la revendication 1 à base d'une résine époxydique, d'un polyuréthanne, d'un aminoplaste, d'une résine acrylique, d'alkydes ou de polyesters, ou d'un mélange de telles résines.

**14.** Produit de revêtement selon la revendication 1 à base d'un polymère vinylique, d'un ester de la cellulose, d'un caoutchouc chloré, d'une résine phénolique, d'un copolymère styrène-butadiène ou d'une huile siccative.

**15.** Composition selon la revendication 1 qui est un lubrifiant.

**16.** Composition lubrifiante selon la revendicatlon 1 qui contient de 0,2 à 2 % en poids d'un composé de formule I tel que défini à la revendication 1.

**17.** Composition lubriiante selon la revendication 15 qui contient un composé de formule IV tel que défini à la revendication 7.

**18.** Procédé pour préparer des composés de formule I selon la revendication 1, procédé caractérisé en ce qu'on chauffe un composé de formule V :

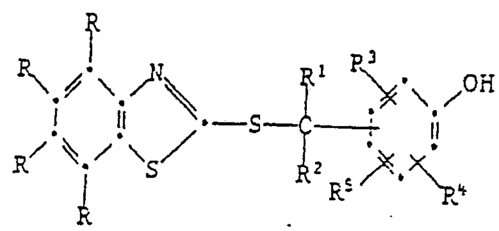

(V)

à une température de 70 à 250° de préférence de 100 à 200°.

**19.** Procédé selon la revendication 18 caractérisé en ce qu'on effectue le chauffage dans un solvant polaire.

**20.** Procédé selon la revendication 18 caractérisé en ce qu'on effectue le chauffage en présence d'un catalyseur basique.

**21.** Procédé pour préparer des composés de formule I selon la revendication 1, procédé caractérisé en ce qu'on fait réagir le mercapto-2 benzothiazole de formule VI :

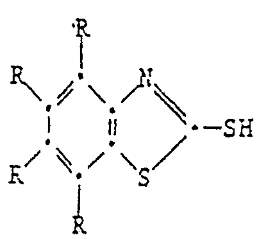

(VI)

et un composé carbonylique

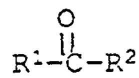

avec un phénol de formule VIII :

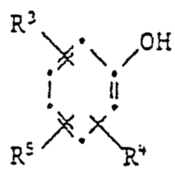

(VIII)

en présence d'un catalyseur basique.

**22.** Procédé selon la revendication 21 caractérisé en ce qu'on effectue la réaction dans un solvant polaire.